(19) Europäisches Patentamt
European Patent Office
Office européen des brevets

(11) EP 1 283 209 A1

(12) **EUROPEAN PATENT APPLICATION**
published in accordance with Art. 158(3) EPC

(43) Date of publication:
**12.02.2003 Bulletin 2003/07**

(21) Application number: **01932147.0**

(22) Date of filing: **18.05.2001**

(51) Int Cl.[7]: **C07D 409/04**, C07D 417/04, A61K 31/4436, A61K 31/427, A61P 5/28, A61P 5/32, A61P 35/00

(86) International application number:
**PCT/JP01/04189**

(87) International publication number:
**WO 01/087878 (22.11.2001 Gazette 2001/47)**

(84) Designated Contracting States:
**AT BE CH CY DE DK ES FI FR GB GR IE IT LI LU MC NL PT SE TR**

(30) Priority: **18.05.2000 JP 2000146579**

(71) Applicant: **DAIICHI PHARMACEUTICAL CO., LTD.**
**Chuo-ku, Tokyo 103-8234 (JP)**

(72) Inventors:
- **SHIMADA, Shin'ichi**
  **Shimotsuga-gun, Tochigi 329-0511 (JP)**
- **NOMOTO, Shin, Erumezongakuenminami 2-303**
  **Kawachi-gun, Tochigi 329-0434 (JP)**
- **OKUE, Masayuki**
  **Shimotsuga-gun, Tochigi 329-0511 (JP)**
- **KIMURA, Ken'ichi**
  **Hanamaki-shi, Iwate 025-0066 (JP)**
- **NAKAMURA, Junji**
  **Kawachi-gun, Tochigi 329-0434 (JP)**
- **IKEDA, Yoshikazu**
  **Shimotsuga-gun, Tochigi 329-0519 (JP)**
- **TAKADA, Takeko**
  **Utsunomiya-shi, Tochigi 321-0917 (JP)**

(74) Representative: **Wakerley, Helen Rachael**
**Reddie & Grose,**
**16 Theobalds Road**
**London WC1X 8PL (GB)**

(54) **NOVEL BENZOTHIOPHENE DERIVATIVES**

(57) The present invention relates to novel benzothiophene derivatives represnted by the following formula (I) or salts thereof, inhibitors of steroid 17α-hydroxylase and/or C17-20 lyase, and pharmaceutical compositions containing the benzothiophene derivative or the salt.

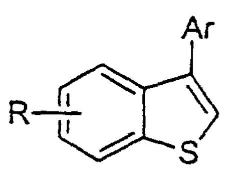

(I)

wherein Ar shows a substituted or unsubstituted aromatic heterocyclic group; R shows a hydroxyl group, lower alkyl group, lower alkyloxy group, halogen atom, carboxyl group, lower alkyloxycarbonyl group, carbamoyl group, amino group, amino group which may be substituted or not substituted with one or more substituents selected from a lower alkyl group and lower acyl group, cyano group, substituted or unsubstituted phenyl group, substituted or unsubstituted phenoxy group, substituted or unsubstituted phenyl lower alkyl group, substituted or unsubstituted phenyl lower alkyloxy group, or substituted or unsubstituted aromatic heterocyclic group.

EP 1 283 209 A1

**Description**

FIELD OF THE INVENTION

The present invention relates to novel benzothiophene derivatives. The present invention also relates to inhibitors of steroid 17α-hydroxylase and/or steroid C17-20 lyase and to pharmaceutical compositions.

BACKGROUND OF THE INVENTION

In the formation of sex steroids in living bodies, 1)C21-steroids, such as a progesterone, are formed from cholesterol, 2) androgenic hormones, such as androstenedione and testosterone, which are C19-steroids, are synthesized from C21-steroids by steroid 17α-hydroxylase and/or steroid C17-20 lyase, and 3)estrogens, such as estrone and estradiol, which are C18-steroids, are synthesized from these C19-steroids as a substrate by aromatase enzymes. All these sex steroids are known to exhibit various activities. If the steroid 17α-hydroxylase and/or steroid C17-20 lyase or aromatase, which are enzymes synthesizing these sex steroids, are inhibited, *in vivo* formation of androgenic hormones and/or estrogens can be controlled. Thus, it is possible to prevent or treat various diseases, in which androgenic hormones or estrogens are involved as an exacerbation factor, such as prostate cancer, prostatic hypertrophy (prostatism), androgenic syndrome (masculinism), andromorphous baldness, breast cancer, mastopathy, uterine cancer, endometriosis, and ovarian cancer.

It is already revealed from numerous findings that reducing the amount of androgenic hormones in blood can treat these diseases relating to androgenic hormones, such as prostate cancer and prostatic hypertrophy. For example, conventionally decrease in the androgenic hormones was brought about by orchidectomy or adrenalectomy. A decrease in the androgenic hormones originating from the gonad gland by the administration of an LH-RH agonist, which is a kind of hypophysis hormone, has been reported recently to exhibit treatment effects.

However, the above-mentioned evisceration is not only psychologically difficult to accept, but also may be accompanied by side effects caused by a decrease of mineral corticoid or gluco-corticoid from the adrenal glands. The administration of an LH-RH agonist only inhibits synthesis of hormones of gonad gland origin and is not expected to decrease hormones originating from other organs, such as the adrenal glands. In addition, a problem of "flare phenomenon" due to a temporary increase of hormones unique to the agonist has been indicated.

On the other hand, although anti-androgenic hormone agents antagonistic to androgenic hormone receptors have been developed, it is reported that the effect of such an agent decreases due to denaturing of the androgenic hormone receptors.

In view of this situation, development of a more effective agent for decreasing androgenic hormones is desired. It is possible to decrease greatly androgenic hormones by inhibiting steroid17α-hydroxylase and/or steroid C17-20 lyase. Therefore, inhibition of these steroids is expected to exhibit high effects in the treatment of various diseases in which the androgenic hormones are involved, such as prostate cancer, prostatic hypertrophy, and masculinization disease. In addition, inhibition of steroid 17α-hydroxylase and/or steroid C17-20 lyase may result in interruption of estrogen synthesis.

Up to the present time, steroid compounds and non-steroid compounds have been known as inhibitors of steroid 17α-hydroxylase and/or steroid C17-20 lyase. Examples include non-steroid compounds such as imidazole derivatives disclosed in Japanese Patent Application (Laid-open) No. 64-85975 andazole derivatives having a condensed three-ring structure disclosed in WO 95/09157. However, because these compounds are not necessarily satisfactory in their effects, development of compounds exhibiting higher activity has been desired.

DISCLOSURE OF THE INVENTION

In view of the above situation, the inventors of the present invention have carried out extensive studies to discover substances inhibiting steroid 17α-hydroxylase and/or steroid C17-20 lyase. As a result, the inventors have found that a certain compound possessing a benzothiophene skeleton exhibits potent inhibitory activity of steroid 17α-hydroxylase and/or steroid C17-20 lyase, as well as of aromatase. Therefore, an object of the present invention is to provide novel benzothiophene derivatives, which inhibit steroid 17α-hydroxylase and/or steroid C17-20 lyase. Another object of the present invention is to provide novel steroid 17α-hydroxylase and/or steroid C17-20 lyase inhibitors or pharmaceutical compositions.

The present invention relates to novel benzothiophene derivatives. The compounds of the present invention exhibit potent inhibitory activity of steroid 17α-hydroxylase and/or steroid C17-20 lyase. They also exhibit inhibitory activity of aromatase. Due to its activity, the compounds of the present invention are useful as preventive and/or therapeutic agents for various diseases, in which androgenic hormones and estrogens are involved, such as prostate cancer, prostatic hypertrophy (prostatism), androgenic syndrome (masclulinizaiton), breast cancer, mastopathy, uterine

cancer, endometriosis, and ovarian cancer.

Specifically, the present invention provides novel benzothiophene derivatives represented by the following formula (I) or salts thereof:

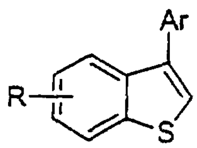

( I )

wherein Ar is a substituted or unsubstituted aromatic heterocyclic group and R is a hydroxyl group, lower alkyl group, lower alkyloxy group, halogen atom, carboxyl group, lower alkyloxycarbonyl group, carbamoyl group, morpholino group, amino group, amino group which may be substituted or not substituted with one or more substituents selected from a lower alkyl group and lower acyl group, cyano group, substituted or unsubstituted phenyl group, substituted or unsubstituted phenoxy group, substituted or unsubstituted phenyl lower alkyl group, substituted or unsubstituted phenyl lower alkyloxy group, or substituted or unsubstituted aromatic heterocyclic group. The lower alkyl group is a linear, branched, or cyclic hydrocarbon having 1-7 carbon atoms, wherein the hydrocarbon may be substituted with a halogen atom, hydroxyl group, alkyloxy group, amino group, amino group which may be substituted or not substituted with one or two substituents selected from a lower alkyl group and lower acyl group, nitro group, or cyano group. The halogen atom is a fluorine atom, chlorine atom, bromine atom, or iodine atom. As a substituent for the substituted phenyl group, substituted phenoxy group, substituted phenyl lower alkyl group, or substituted phenyl lower alkyloxy group represented by R, or for the substituted aromatic heterocyclic group represented by Ar or R, a hydroxyl group, lower alkyl group, lower alkylcarbonyl group, lower alkyloxy group, lower alkylthio group, halogen atom, carboxyl group, lower alkyloxycarbonyl group, carbamoyl group, amino group, amino group which may be substituted or not substituted with one or two substituents selected from a lower alkyl group and a lower acyl group, nitro group, and cyano group can be given, wherein the number of substituent may be 1-3; further, the two substituents in combination may form a lower alkylenedioxy group.

As examples of the aromatic heterocyclic group in the compound of the present invention, heterocyclic groups containing a nitrogen atom and/or sulfur atom as the heteroatom, such as a pyridyl group, thienyl group, and thiazole group, can be given.

The following compounds can be given as specific examples of the novel benzothiophene derivatives represented by the formula (I) of the present invention:

(1) 4-[6-methoxybenzo[b]thiophen-3-yl]pyridine,
(2) 3-[6-methoxybenzo[b]thiophen-3-yl]pyridine,
(3) 2-[6-methoxybenzo[b]thiophen-3-yl]pyridine,
(4) 2-[6-methoxybenzo[b]thiophen-3-yl]thiazole,
(5) 5-[6-methoxybenzo[b]thiophen-3-yl]-2,4-dimethylthiazole,
(6) 3-(4-pyridyl)benzo[b]thiophen-6-ol,
(7) 4-[6-(4-methoxyphenyl)benzo[b]thiophen-3-yl]pyridine,
(8) 3-(3-pyridyl)benzo[b]thiophen-6-ol,
(9) 3-(3-pyridyl)benzo[b]thiophen-6-yl=acetate,
(10) 3-(3-pyridyl)benzo[b]thiophen-6-yl=benzoate,
(11) 3-(6-benzyloxybenzo[b]thiophen-3-yl)pyridine,
(12) 3-(6-isopropyloxybenzo[b]thiophen-3-yl)pyridine,
(13) 3-[6-(4-fluorophenyl)benzo[b]thiophen-3-yl]pyridine,
(14) 3-[6-(3-fluorophenyl)benzo[b]thiophen-3-yl]pyridine,
(15) 3-(6-phenylbenzo[b]thiophen-3-yl)pyridine,
(16) 3-[6-(3-methoxyphenyl)benzo[b]thiophen-3-yl]pyridine,
(17) 3-[3-(3-pyridyl)benzo[b]thiophen-6-yl]phenol,
(18) 3-[6-(4-methoxyphenyl)benzo[b]thiophen-3-yl]pyridine,
(19) 4-[3-(3-pyridyl]benzo[b]thiophen-6-yl]phenol,
(20) 3-[6-(3-pyridyl)benzo[b]thiophen-3-yl]pyridine,
(21) 3-[6-(3,4-dimethoxyphenyl)benzo[b]thiophen-3-yl] pyridine,
(22) 4-[3-(3-pyridyl)benzo[b]thiophen-6-yl]benzene-1,2-diol,
(23) 3-[3-(3-pyridyl)benzo[b]thiophen-6-yl]phenylamine,

(24) 3-[3-(3-pyridyl)benzo[b]thiophen-6-yl]acetylamino benzene,
(25) 3-(6-cyclopentyloxybenzo[b]thiophen-3-yl)pyridine,
(26) 3-[6-(3-methoxyphenoxy)benzo[b]thiophen-3-yl]pyridine,
(27) 3-(6-methylbenzo[b]thiophen-3-yl)pyridine,
(28) 3-(6-bromobenzo[b]thiophen-3-yl)pyridine,
(29) 3-(4-bromobenzo[b]thiophen-3-yl)pyridine,
(30) 3-(6-cyclobutyloxybenzo[b]thiophen-3-yl)pyridine,
(31) 3-[6-(3-thienyl]benzo[b]thiophen-3-yl]pyridine,
(32) 3-[6-(3-furyl)benzo[b]thiophen-3-yl]pyridine,
(33) 3-[6-(2-thienyl)benzo[b]thiophen-3-yl]pyridine,
(34) 3-[6-(2-furyl)benzo[b]thiophen-3-yl]pyridine,
(35) 3-(6-hexyloxybenzo[b]thiophen-3-yl)pyridine,
(36) 3-(6-amyloxybenzo[b]thiophen-3-yl)pyridine,
(37) 3-(6-butyloxybenzo[b]thiophen-3-yl)pyridine,
(38) 1-{2-[3-(3-pyridyl)benzo[b]thiophen-6-yl]phenyl}-3-ethanone,
(39) 1-{3-[3-(3-pyridyl)benzo[b]thiophen-6-yl]phenyl}-3-ethanone,
(40) 3-(6-isobutyloxybenzo[b]thiophen-3-yl)pyridine,
(41) 3-(6-propyloxybenzo[b]thiophen-3-yl)pyridine,
(42) 3-[6-(1,3-benzodioxol-5-yl)benzo[b]thiophen-3-yl] pyridine,
(43) 3-(6-ethyloxybenzo[b]thiophen-3-yl)pyridine,
(44) 1-{4-[3-(3-pyridyl)benzo[b]thiophen-6-yl]phenyl}-1-ethanone,
(45) 3-[6-(N,N-dimethyl-2-aminoethyloxy)benzo[b]thiophen-3-yl]pyridine,
(46) 3-(6-isobutylbenzo[b]thiophen-3-yl)pyridine,
(47) 3-[6-(4-methylthiophenyl)benzo[b]thiophen-3-yl]pyridine,
(48) 3-{[6-(2-trifluoromethyl)phenyl]benzo[b]thiophen-3-yl}pyridine,
(49) isopropyl{3-[3-(3-pyridyl)benzo[b]thiophen-6-yl] phenyl}amine,
(50) 3-(6-cyclohexylbenzo[b]thiophen-3-yl)pyridine,
(51) 3-[6-(3-isopropyloxyphenyl)benzo[b]thiophen-3-yl] pyridine,
(52) 3-(6-propylbenzo[b]thiophen-3-yl)pyridine,
(53) 3-[6-(3-nitrophenyl)benzo[b]thiophen-3-yl]pyridine,
(54) isopropyl[3- (3-pyridyl)benzo[b]thiophen-6-yl]amine,
(55) 4-[3-(3-pyridyl)benzo[b]thiophen-6-yl]morpholine,
(56) Nl-[3-(3-pyridyl)benzo[b]thiophen-6-yl]acetamide.

In addition to the above-mentioned compounds, salts formed from these compounds and an acid or base, are included in the compounds of the present invention. As acid addition salts, salts with a mineral acid, such as a hydrochloride, hydrobromide, hydroiodide, sulfate, nitrate, and phosphate, and salts with an organic acid, such as a formate, acetate, proprionate, oxalate, malonate, succinate, fumarate, maleate, lactate, malate, citrate, tartrate, carbonate, picrate, methanesulfonate, and glutamate can be given. As salts with a base, inorganic salts, such as a sodium salt, potassium salt, magnesium salt, calcium salt, and aluminium salt; organic salts, such as a lower alkylamine salt and lower alcoholic amine; salts with a basic amino acid such as lysine salts, argine salts, and ornithine salts; ammonium salts; and the like can be given. In addition, the compound of the present invention may form a hydrate or a solvate with a lower alcohol and the like.

The compounds (I) of the present invention can be prepared by the method shown by the following reaction formula, for example. In the following schematic reaction formula for the preparation of the compounds of the present invention, each symbol used in the compounds is the same as those previously described.

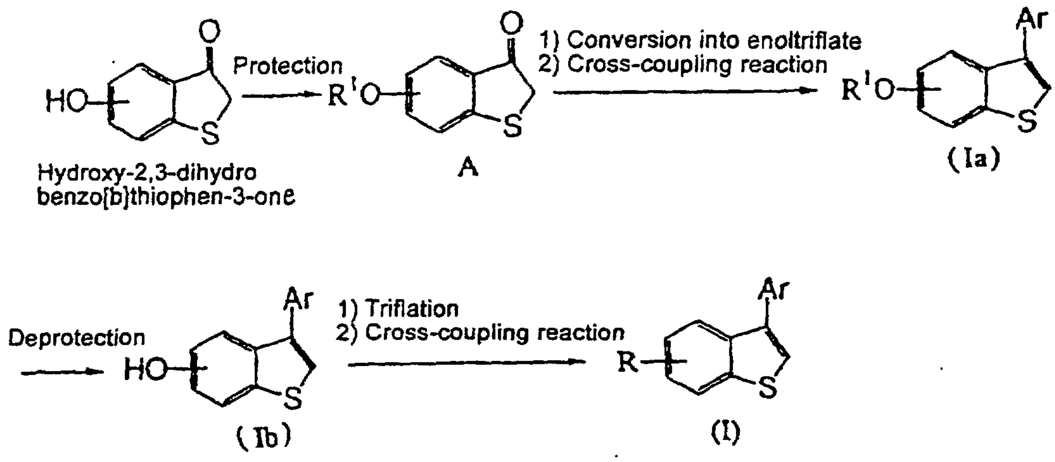


Hydroxy-2,3-dihydro benzo[b]thiophen-3-one A (Ia) (Ib) (I)

The hydroxyl group in hydroxy-2,3-dihydrobenzo[b] thiophen-3-one is protected to prepare a compound A. Then, after converting into an enoltriflate, compound (Ia) is prepared by a cross-coupling reaction using various types of aryl boronic acid, aryl boronic acid ester or borane derivative, and a transition metal catalyst. The protective group ($R^1$) is removed from compound (Ia) by a deprotecting reaction to prepare compound (Ib), which is then converted into an enoltriflate, followed by a cross-coupling reaction using various types of aryl boronic acid, aryl boronic acid ester, borane derivative, or an alkyl zinc halide, and a transition metal catalyst, thereby obtaining the objective compound (I). Here, it should be noted that compounds (Ia) and (Ib) are included within the compound (I) of the present invention. The group $R^1$ in the above formula represents a protective group for the hydroxyl group. As required, a substituent in the phenyl group or aromatic heterocyclic group represented by R is modified to obtain the objective compound. Here, example modifications of the substituent include dealkylation of an alkyl ether, acylation or alkylation of a hydroxyl group or amino group, and the like.

In an alternative method of obtaining compound (Ib), compound (A) is converted into an enoltriflate, followed by a cross-coupling reaction using a boronating agent such as a tetra-alcoholate diboronic acid (bis(pinacolate) diboronic acid, for example) and a transition metal catalyst, to obtain a benzo[b]thiophene-3-boronic acid ester derivative. This compound is then subjected to a cross coupling reaction using a sulfate derivative of various halogenated Ar or hydroxy Ar (using Cl, Br, or I as the halogen, and an ester of methanesulfonic acid or trifluoromethane sulfonic acid as the sulfate) and a transition metal catalyst to prepare compound (Ib). The reaction formula is shown below.

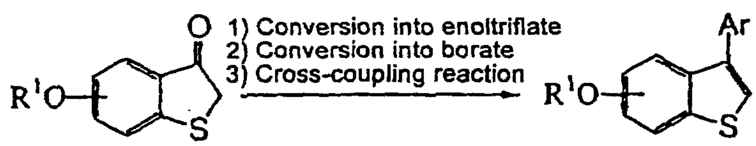


A (Ia)

Compound (Ia) can also be obtained by a condensation and cyclization reaction of a hydroxythiophenol derivative D, the hydroxyl group of which is protected by a protective group, with various bromoacetyl derivatives E. Compound (Ia) is then converted into compound (Ib) by a deprotecting reaction. Compound (Ib) is converted into an enoltriflate, followed by a cross-coupling reaction using various aryl boronic acid, aryl boronic acid ester, borane derivative, or alkyl zinc halide, thereby obtaining the objective compound (I). The objective compound (I) may also be obtained by modifying the hydroxyl group of compound (Ib). Further, the objective compound (I) can also optionally be obtained by modifying the substituent in the phenyl group or aromatic heterocyclic group represented by R. The reaction formula is shown below.

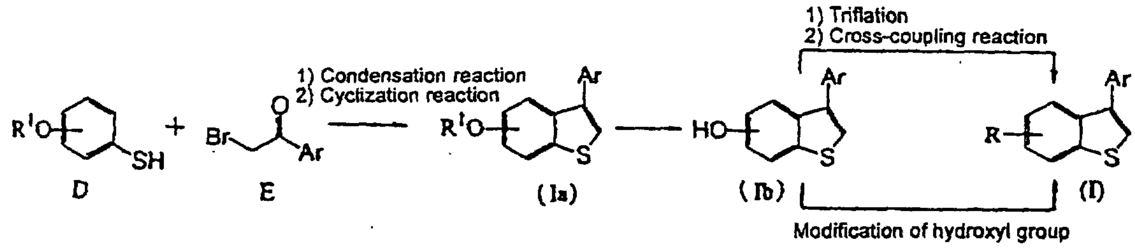


In the reactions shown by the above three chemical reaction formulae, the raw material compound and the intermediates may be either a free compound or a salt, similar to the compound (I). In addition, the reaction mixture may be subjected to the reaction either as is or after isolation using a known method.

The amino group, carboxyl group, and hydroxyl group of the compounds or their salts submitted to the reactions, which are uninvolved in the reactions, may be protected using a protective group. Known methods, such as that described in "PROTECTIVE GROUPS in ORGANIC SYNTHESIS" by T. W. Greene, P. G. M. Wuts, publishedby Wiley-Interscience (1999), and methods conforming to this method, may be applied to the addition or removal of the protective groups. As a protective group, ethers such as methyl ether, methoxymethyl ether, ethyl ether, 1-ethoxyethyl ether, phenacyl, and tetrahydropyranyl; silyl ethers such as trimethylsilyl ether and t-butyldimethylsilyl ether; and esters such as a formate and acetate may be used.

Usually, an organic solvent not affecting the reaction is used as a solvent. Examples of organic solvents not adversely affecting the reaction are: saturated hydrocarbons such as hexane and pentane; amides such as N,N-dimethylformamide (DMF) and N,N-dimethylacetamide; halogenated hydrocarbons such as dichloromethane and chloroform; ethers such as diethyl ether, dioxane, andtetrahydrofuran (THF) ; esters such as methyl acetate and ethyl acetate; alcohols such as methanol, ethanol, 1-propanol, 2-propanol, 2-methyl-2-propanol, and 1-butanol; nitriles such as acetonitrile and propionitrile; nitroalkanes such as nitromethane and nitroethane; and aromatic hydrocarbons such as benzene, toluene, and pyridine. These solvents may be used either individually or in combination of two or more at appropriate proportion.

When a base is used in the condensation reaction, triflatization reaction, and cross-coupling reaction, said base may include an alkali metal such as lithium hydroxide, sodium hydroxide, potassium hydroxide, cesium carbonate, sodium hydrogen carbonate, potassium hydrogen carbonate, trisodium phosphate, tripotassium phosphate, sodium acetate, and potassium acetate; an alkali metal hydrides such as sodium hydride, potassium hydride; amines such as diisopropyl ethyl amine, 2,6-lutidine, 2,6-di-t-butylpyridine, 2,6-di-t-butyl -4-methylpyridine, and triethylamine; and the like.

When an acid is used in the cyclization reaction or deprotection reaction, said acid may include a mineral acid such as hydrochloric acid, hydrobromic acid, sulfuric acid, phosphoric acid, and polyphosphoric acid; an organic acid such as trifluoroacetic acid, p-toluene sulfonic acid, and methanesulfonic acid; Lewis acid such as zinc chloride, tin chloride, boron trifluoride diethyl ether complex, aluminium chloride, and titanium tetrachloride; and the like.

Example transition metal catalysts used in the cross-coupling reaction (indicating homo or hetero nuclear bond-formation reaction represented by Heck reaction, Suzuki reaction, Ullmann reaction, for example) are palladium, nickel, and copper, each having 0 to 2 valence. These metals may form a complex with triphenylphosphine, dibenzylidene acetone, bis-diphenyl phosphinoferrocene, and the like. The cross-coupling reaction is usually carried out at a temperature of -80 to 100°C, and preferably 0 to 100°C, for usually about 5 minutes to about 5 days, and preferably 30 minutes to 20 hours.

The compounds and the salt thereof of the present invention can be orally or parenterally administered safely to human beings and animals as pharmaceuticals. Suitable means for parenteral administration are intravenous injection, intramuscular injection, hypodermic injection, intraperitoneal injection, transdermal (percutaneous) administration, transpulmonary administration, pernasal administration, intestinal administration, intraoral administration, transmucosal administration, and the like. Preparations for these purposes are used. Specific examples of the preparations may inculude injection, suppositories, aerosol agents, percutaneous absorption tapes, and the like. Oral administration preparations include, for example, tablets (including sugar-coated tablets, coated tablets, buccal tablets), powder, capsules (including soft capsules), granules (including coated granules), pilules, troches, and liquid preparations, as well as their pharmaceutically acceptable sustained release preparations. Liquid preparations for oral administration include suspension, emulsion, syrup, (including dry syrup), and elixir.

These preparations are formulated according to known methods of making pharmaceutical preparations using pharmaceutically acceptable carriers, vehicles (excepients), disintegrators, lubricants, coloring agents, and the like for dosing as a pharmaceutical composition. Example carriers and vehicles are lactose, glucose, saccharose, mannitol,

potato starch, cornstarch, calcium carbonate, calcium phosphate, calcium sulfate, crystalline cellulose, powdered glycyrrhiza, and powdered gentian. Example binders are starch, Tragacanth rubber, gelatin, syrup, polyvinyl alcohol, polyvinyl ether, polyvinyl pyrrolidone, hydroxypropyl cellulose, methylcellulose, ethyl cellulose, and carboxymethyl cellulose. Suitable disintegrators are starch, agar, gelatin powder, sodium carboxymethyl cellulose, calcium carboxymethyl cellulose, crystalline cellulose, calcium carbonate, sodium hydrogencarbonate, and sodium alginate. Example lubricants are magnesium stearate, talc, hydrogenated vegetable oils, macrogol, and the like. As coloring agents, any pharmaceutically acceptable coloring agents may be used.

Tablets and granules may be optionally coated with saccharose, gelatin, purified shellac, glycerol, sorbitol, ethylcellulose, hydroxypropyl cellulose, hydroxypropylmethyl cellulose, polyvinyl pyrrolidone, phthalic acid cellulose acetate, hydroxypropyl methylcellulose phthalate, methyl methacrylate, methacrylic acid polymer, and the like. Further, tablets and granules may be coated with layer using two or more coating agents above. Capsules made of a compound such as ethylcellulose and gelatin may also be used. When preparing a composition for injection, a pH adjusting agent, buffering agent, stabilizer, solubilizer, and the like, may optionally be added to the principal component according to conventional methods.

When the compound of the present invention is administered to a patient, the dose varies depending on the conditions such as a degree of symptom, age of the patient, health conditions, and body weight. A daily dose per adult for oral or non-oral administration may be in the range of 1-1000 mg, preferably 50-200 mg, and once or more per day, but not limited to this range.

## PREFERRED EMBODIMENTS TO CARRY OUT THE INVENTION

The present invention will now be described in more detail by way of examples, which are given for the purpose of explanation and should not be construed as limiting the present invention.

### Example 1

Preparation of 4-[6-methoxybenzo[b]thiophen-3-yl]pyridine hydrochloride

3-methoxybenzenethiol (0.60 ml, 4.836 mmol) was added to a solution of potassium hydroxide (0.74g, 11.21 mmol) in a mixture of water (6 ml) and ethanol (9 ml) under cooling with ice. After the addition of 2-bromo-1-pyridin-4-yl-ethanone hydrobromide (1.70 g, 6.051 mmol; H. Erlenmeyen et al., Helv. Chim. Acta., 31, 1142 (1948)), the mixture was stirred for 3.5 hours at room temperature. After removing ethanol by evaporation under reduced pressure, the residue was extracted with diethyl ether (100 ml). The organic layer was washed with water, and then with saturated brine (sodium chloride solution), dried with anhydrous magnesium sulfate, then the solvent was evaporated under reduced pressure to obtain crude 2-(3-methoxyphenylsulfanyl)-1-pyridin-4-yl ethanone (700 mg, 56%).

$^1$H-NMR($CDCl_3$) $\delta$: 3.75(s, 3H) , 4.19(s, 2H), 6.77(dd, J=1.8, 8.6Hz, 1H), 6.88(dd, J=1.8, 2.4, 1H), 6.91(d, J=7.9Hz, 1H), 7.18(t, J=7.9Hz, 1H), 7.67(d, J=6.1Hz, 2H), 8.77(d, J=6.1Hz, 2H).

The crude 2-(3-methoxy phenylsulfanyl)-1-pyridin-4-yl ethanone (700 mg, 2.699 mmol) obtained in the above reaction was dissolved in borontrifluoride-diethyl ether complex (30 ml, 0.2367 mol). The solution was stirred for 16.5 hours under a nitrogen stream at room temperature. The reaction solution was carefully poured into ice water and 5N sodium hydroxide aqueous solution was added under cooling with ice to obtain a pH 8 water layer. The product was extracted with diethyl ether (150 ml). The organic layer was washed with water, then with saturated brine and dried with anhydrous magnesium sulfate. The solvent was evaporated under reduced pressure and the resulting residue was charged to basic silica gel column chromatography (hexane:ethyl acetate = 17:3) to obtain a colorless oily product of 4-[6-methoxybenzo[b]thiophen-3-yl]pyridine (420 mg, 36%). The product was dissolved in diethyl ether (3 ml). 1N hydrogen chloride-diethyl ether solution (2 ml, 2 mmol) was added to the diethyl ether solution under ice cooling to obtain a precipitate. The precipitate was filtered to obtain a white powder of 4-[6-methoxybenzo[b]thiophen-3-yl]pyridine hydrochloride (425 mg, 32%).

$^1$H-NNR(DMSO-$d_6$) $\delta$:3.86 (s, 3H), 7.15 (dd, J=1.8, 8.8Hz, 1H), 7.76 (d, J=2.4Hz, 1H), 7.95(d,J=8.8Hz, 1H), 8.21(d, J=6.7Hz, 2H), 8.30 (s, 1H), 8.92(d, J=6.7Hz, 2H).

IR(KBr):1630, 1602, 1524, 1505, 1488, 1469, 1440, 1235, 1041, 838, 795$cm^{-1}$.

Melting point: 236.5°C -239.0°C

### Example 2

Preparation of 3-[6-methoxybenzo[b]thiophen-3-yl]pyridine hydrochloride

3-methoxybenzenethiol (0.60 ml, 4.836 mmol) was added to a solution of potassium hydroxide (0.74 g, 11.21

mmol) in a mixture of water (6 ml) and ethanol (9 ml) under cooling with ice. After the further addition of 2-bromo-1-pyridin-3-yl ethanone hydrobromide (1.70 g, 6.051 mmol; A. T. Nielsen et al., Het. Chem., 6, 891(1961)), the mixture was stirred for 3.5 hours at room temperature. After removing ethanol by evaporation under reduced pressure, the product was extracted with diethyl ether (100 ml). The organic layer was washed with water, then with saturated brine, dried with anhydrous magnesium sulfate, then the solvent was evaporated under reduced pressure to obtain crude 2-(3-methoxyphenylsulfanyl)-1-pyridin-3-yl ethanone (1.27 g). $^1$H-NMR($CDCl_3$) δ:3.75 (s, 3H), 4.22(s, 2H), 6.75(ddd, J=0.9, 2.5, 8.2Hz,1H), 6.89(dd, J=1.8, 2.4Hz, 1H), 6.93(ddd, J=0.9, 1.8, 7.9Hz, 1H), 7.18(dd, J=7.9, 8.2Hz, 1H), 7.39 (ddd, J=0. 9, 4.9, 7.9Hz, 1H) , 8.18 (ddd, J=1.8, 2.1, 7.9Hz, 1H), 8.76(dd, J=1.5, 4.9Hz, 1H), 9,12(dd, J=0.9, 2.1Hz, 1H).

The crude 2-(3-methoxy phenylsulfanyl)-1-pyridin-3-yl ethanone (1.27g, 4.836 mmol) obtained in the above reaction was dissolved in borontrifluoride-diethyl ether complex (30 ml, 0.2367 mol). The solution was stirred for 16.5 hours under nitrogen stream at room temperature. The reaction solution was carefully poured into ice water and 5N sodium hydroxide aqueous solution was added under cooling with ice to obtain a pH 8 water layer. The solution was extracted with diethyl ether (150 ml). The organic layer was washed with water, then with saturated brine and dried with anhydrous magnesium sulfate. The solvent was evaporated under reduced pressure and the resulting residue was charged to silica gel column chromatography (hexane:ethyl acetate = 17:3) to obtain a colorless oily product of 3-[6-methoxybenzo[b]thiophen-3-yl]pyridine (420 mg, 36%). The colorless oily product was dissolved in diethyl ether (3 ml). 1N hydrogen chloride-diethyl ether solution (2 ml, 2 mmol) was added to the solution under ice cooling to obtain a precipitate. The precipitate was filtered to obtain a white powder of 3-[6-methoxybenzo[b]thiophen-3-yl]pyridine hydrochloride (440 mg, 33%).
$^1$H-NMR(DMSO-$d_6$) δ: 3.85(s, 3H), 7.10(dd, J=2.4, 8.8Hz, 1H), 7.71 (d, J=2.4Hz, 1H), 7.82 (d, J=8.8Hz, 1H), 8.01 (s, 1H), 8.06 (dd, J=5.6, 7.8Hz, 1H), 8.69(dt, J=1.6, 7.8Hz, 1H), 8.88(dd, J=1.2, 5.5Hz, 1H), 9.11(d, J=2.1Hz, 1H).
IR(KBr):1600, 1559, 1453, 1236, 1057, 1026, 796cm$^{-1}$.
Melting point: 222.0-223.5°C

Example 3

Preparation of 2-[6-methoxybenzo[b]thiophen-3-yl]pyridine hydrochloride

3-methoxybenzenethiol (0.60 ml, 4.836 mmol) was added to a solution of potassiumhydroxide (0.74g, 11.21mmol) in a mixture of water (6 ml) and ethanol (9 ml) under cooling with ice. After the further addition of 2-bromo-1-pyridin-2-yl ethanone hydrobromide (1.70 g, 6.051 mmol; A. T. Nielsen et al., J. Het. Chem. 6, 891(1961)), the mixture was stirred for 3.5 hours at room temperature. After removing ethanol by vacuum evaporation, the residue was extracted with diethyl ether (100 ml). The organic layer was washed with water, then with saturated brine, dried with anhydrous magnesium sulfate, then the solvent was evaporated under reduced pressure to obtain crude 2-(3-methoxyphenylsulfanyl)-1-pyridin-2-yl ethanone (1.26g).
$^1$H-NMR($CDCl_3$) δ: 3.76(s, 3H), 4.55 (s, 2H) , 6.71(dd, J-2.4, 8.1Hz, 1H), 6.95-6.97(m, 2H), 7.04(m, 1H), 7.16(t, J=8.1Hz, 1H), 7.45-7.48(m, 1H), 7.83(dt, J=1.8, 7.9Hz, 1H), 8,04(d, J=7.9Hz, 1H), 8.66(d, J=4.6Hz, 1H).

The crude 2-(3-methoxy phenylsulfanyl)-1-pyridin-2-yl ethanone (1.26 g, 4.836 mmol) obtained in the above reaction was dissolved in borontrifluoride-diethyl ether complex (30 ml, 0.2367mol). The solution was stirred for 16 hours under nitrogen stream at room temperature. The reaction solution was carefully poured into ice water and 5N sodium hydroxide aqueous solution was added under ice cooling to obtain a pH 8 water layer. The product was extracted with diethyl ether (150 ml). The organic layer was washed with water, then with saturated brine and dried with anhydrous magnesium sulfate. The solvent was evaporated under reduced pressure and the residue was charged to silica gel column chromatography (hexane:ethyl acetate = 93:7) to obtain a colorless oily product of 2-[6-methoxybenzo[b]thiophen-3-yl]pyridine (180 mg, 15%). The colorless oily product was dissolved in ethanol (1 ml). 1N hydrogen chloride-diethyl ether solution (1ml, 1 mmol) was added to the solution under ice cooling. The mixture was diluted with diethyl ether to obtain a precipitate. The precipitate was filtered to obtain a white powder of 2-[6-methoxybenzo[b]thiophen-3-yl]pyridine hydrochloride (190 mg, 14%).
$^1$H-NMR(DMSO-$d_6$) δ: 3.85(s, 3H) , 7.11(dd, J-2.4, 9.1Hz, 1H), 7.70-7.73(m,2H), 8.12(d, J=2.3Hz, 1H), 8.25-8.31(m, 3H), 8.81 (d, J=4.3Hz, 1H).
IR(KBr):1603, 1541, 1453, 1349, 1273, 1234, 1056, 864, 801, 776cm$^{-1}$.
Melting point: 205.5-207.0°C (decomposed)

Example 4

Preparation of 2-[6-methoxyben2o[b]thiophen-3-yl]thiazole

3-methoxybenzenethiol (0.60 ml, 4.836 mmol) was added to a solution of potassium hydroxide (0.74 g, 11.21

mmol) in a mixture of water (6 ml) and ethanol (9 ml) under cooling with ice. After the further addition of 2-bromo-1-thiazol-2-yl-ethanone hydrobromide (1.53 g, 5.332 mmol), the mixture was stirred for 3 hours at room temperature. After removing ethanol by vacuum evaporation, the residue was extracted with diethyl ether (100 ml). The organic layer was washed with water, then with saturated brine, dried with anhydrous magnesium sulfate, then the solvent was evaporated under reduced pressure to obtain crude 2-(3-methoxyphenylsulfanyl)-1-thiazol-2-yl ethanone (1.29 g).
$^1$H-NMR($CDCl_3$) δ: 3.76(s, 3H) , 4.43(s, 2H) , 6.74(dd, J=2.4, 8.2Hz, 1H), 6.97-6.99(m, 2H), 7.17(t, J=7.9Hz, 1H), 7.70 (d, J=3.1Hz, 1H), 8.01(d, J=3.1Hz, 1H).

The crude 2-(3-methoxyphenylsulfanyl)-1-thiazol-2-yl ethanone (1.29 g, 4.836 mmol) obtained in the above reaction was dissolved in borontrifluoride-diethyl ether complex (30 ml, 0.2367mol). The solution was stirred for 16 hours under nitrogen stream at room temperature. The reaction solution was carefully poured into ice water and 5N sodium hydroxide aqueous solution was added under ice cooling to obtain a pH 8 water layer. The product was extracted with diethyl ether (150 ml). The organic layer was washed with water, then with saturated brine and dried with anhydrous magnesium sulfate. The solvent was evaporated under reduced pressure and the residue was charged to silica gel column chromatography (hexane:ethyl acetate = 2:1). Awhite powder of 2-[6-methoxybenzo[b]thiophen-3-yl]thiazole (780 mg, 65 %) was obtained from the eluate by recrystallizing in ethyl acetate-hexane.
$^1$H-NMR($CDCl_3$) δ: 3.88(s, 3H), 7.10(dd, J=2.4, 8.8Hz, 1H), 7.31(d, J=3.3Hz, 1H), 7.33 (d, J=2.4Hz, 1H), 7.78 (s, 1H), 7.90 (d, J=3.3Hz, 1H), 8.59(d, J=8.8Hz, 1H).
IR(KBr):1604, 1533, 1475, 1268, 1228, 1049, 847, 806, 770, 714cm$^{-1}$.
Melting point: 91.0-91.5°C

Example 5

Preparation of 5-[6-methoxybenzo[b]thiophen-3-yl]-2,4-dimethylthiazole

3-methoxybenzenethiol (0.40 ml, 3.224 mmol) was added to a solution of potassium hydroxide (0.49g, 7.423mmol) in a mixture of water (4 ml) and ethanol (9 ml) under cooling with ice. After the further addition of 2-bromo-1-(2,4- dimethylthiazol-5-yl) ethanone hydrobromide (1.10 g, 3.492 mmol), the mixture was stirred for 3 hours at room temperature. After removing ethanol by vacuum evaporation, the residue was extracted with diethyl ether (100 ml). The organic layer was washed with water, then with saturated brine, dried with anhydrous magnesium sulfate, then the solvent was evaporated under reduced pressure to obtain crude 1-(2,4-dimethylthiazol-5-yl)-2-(3-methoxyphenylsulfanyl) ethanone (0.94 g, 99%).
$^1$H-NMR($CDCl_3$) δ: 2.66(s, 3H), 2.68(s, 3H), 3.76(s, 3H), 4,00(s, 2H), 6.74(dd, J=2.4, 8.2Hz, 1H), 6.91(t, J=2.1Hz, 1H), 6.93(d, J=7.5Hz, 1H), 7.18(dd, J=7.9, 8.2Hz, 1H).

The crude 1-(2,4-dimethylthiazol-5-yl)-2-(3-methoxyphenylsulfanyl)ethanone (0.94 g, 99%) obtained in the above reaction was dissolved in a borontrifluoride-diethyl ether complex (20 ml, 0.1578 mol). The solution was stirred for 16 hours under nitrogen stream at room temperature, then for a further 7.5 hours at 50°C. After cooling, the reaction solution was carefully poured into ice water and 5N sodium hydroxide aqueous solution was added under ice cooling to obtain a pH 8 water layer. The product was extracted with diethyl ether (150 ml). The organic layer was washed with water, then with saturated brine and dried with anhydrous magnesium sulfate. The solvent was evaporated under reduced pressure and the residue was charged to silica gel column chromatography (hexane:ethyl acetate=17:3). A white powder of 5-[6-methoxybenzo[b]thiophen-3-yl]-2,4-dimethylthiazole (256 mg, 29%) was obtained from the eluate by recrystallizing in ethyl acetate-hexane.
$^1$H-NMR($CDCl_3$) δ: 2.34 (s, 3H), 2.71(s, 3H), 3.87(s, 3H), 7.01(dd, J=2.4, 8.8Hz, 1H), 7.22(s, 1H), 7.34(d, J=2.4Hz, 1H), 7.57(d, J=8.8Hz, 1H).
IR(KBr):1600, 1500, 1470, 1434, 1372, 1330, 1266, 1233, 1198, 1048, 1023, 917cm$^{-1}$.
Melting point: 105.0-106.0°C

Example 6

Preparation of 3-(4-pyridyl)benzo[b]thiophen-6-ol

47% hydrobromide solution (9.0 ml) was added to the 4-[6-methoxybenzo[b]thiophen-3-yl]pyridine (260 mg, 1.077 mmol) obtained in Example 1, and the mixture was refluxed for 2 hours. The reaction mixture was neutralized with a 5N sodium hydroxide aqueous solution and saturated sodium bicarbonate solution. The precipitate obtained was filtrated to obtain a white powder of 3-(4-pyridyl)benzo[b]thiophen-6-ol (200 mg, 82%).
$^1$H-NMR(DMSO-$d_6$) δ: 6.96(dd, J=2.4, 8.8Hz, 1H), 7.38(d, J=2.4Hz, 1H), 7.63(d, J=6.0Hz, 2H), 7.77(d, J=8.8Hz, 1H), 7.79(s, 1H), 8.67(d, J=6.0Hz, 2H), 9.76(s, 1H).
IR(KBr) :3100-2600, 1602, 1465, 1249, 824cm$^{-1}$.

Melting point: 246.0-250.0 °C

Example 7

Preparation of 4-[6-(4-methoxyphenyl)benzo[b]thiophen-3-yl]pyridine

Anhydrous trifluoromethane sulfonic acid ($Tf_2O$; 0.14 ml, 0.8322 mmol) was added to a suspension of 3-(4-pyridyl)benzo[b] thiophen-6-ol (164 mg, 0.7216 mmol) obtained in Example 6 and 2,6-di-t-butyl-4-methylpyridine (180 mg, 0.8766 mmol) in dichloromethane (5 ml) under cooling with ice. The mixture was warmed to room temperature and stirred for 2 hours. The reaction mixture was concentrated under reduced pressure. The resulting residue was diluted with ethyl acetate. The organic layer was washed with 5% citric acid aqueous solution, saturated aqueous solution of sodium bicarbonate, water, and then saturated brine, sequentially, and dried with anhydrous magnesium sulfate. The solvent was evaporated under reduced pressure. The residue obtained was submitted to silica gel column chromatography (hexane:ethyl acetate = 17 : 3) to obtain a colorless oily product of 3- (4-pyridyl)benzo[b]thiophen-6-yl=trifluoromethane sulfonate (247 mg, 95%).
$^1$H-NMR($CDCl_3$) δ: 7.33(dd, J=2.4, 8.9Hz, 1H), 7.46(d, J=6.0Hz, 2H), 7.65(s, 1H), 7.85(d, J=2.2Hz, 1H), 7.93(d, J=8.9Hz, 1H), 8.73(d, J=6.0Hz, 2H).

Tetrakistriphenylphosphine palladium (0) (38 mg, 0.03288 mmol) was added to a tetrahydrofuran (THF) suspension (10.0 ml) of 3-(4-pyridyl)benzo[b]thiophen-6-yl=trifluoromethane sulfonate (236 mg, 0.6567 mmol), 4-methoxyphenyl borinic acid (220 mg, 1.447 mmol), and tripotassium phosphate (420 mg, 1.978 mmol). The mixture was stirred for 2 days at 90°C, then cooled to room temperature. After the addition of 2N sodium hydroxide aqueous solution (0.1 ml) and 30% hydrogen peroxide aqueous solution (0.1 ml), the mixture was stirred for 1 hour at the same temperature. After diluting the reaction mixture with ether, the mixture was washed with water and saturated brine, and dried with anhydrous magnesium sulfate. The solvent was evaporated under reduced pressure. The resulting residue was submitted to silica gel column chromatography (hexane:ethyl acetate = 9:1). A white powder of 4-[6-(4-methoxyphenyl) benzo[b] thiophen-3-yl]pyridine (100 mg, 48%) was obtained from the eluate by crystallizing in ethyl acetate-hexane.
$^1$H-NMR($CDCl_3$) δ: 3.86(s, 3H), 7.00(d, J=8.9Hz, 2H), 7.52-7.53 (m, 3H), 7.60(d, J=8.9Hz, 2H), 7.62(dd, J=1.8, 8.5Hz, 1H), 7.94(d, J=8.5Hz, 1H), 8.07(d, J=1.5Hz, 1H), 8.71(d, J=6.1Hz, 2H).
IR(KBr):2832, 1597, 1512, 1247, 817cm$^{-1}$.
Melting point: 157.0-158.5°C

Example 8

Preparation of 3-(3-pyridyl)benzo[b]thiophen-6-ol

47% hydrobromide solution (80 ml) was added to 3-[6-methoxybenzo[b]thiophen-3-yl]pyridine (2.16 g, 8.951 mmol) obtained in Example 2, and the mixture was refluxed for 1 hour. The reaction mixture was neutralized with 5N sodium hydroxide aqueous solution and saturated sodium bicarbonate solution. The precipitate obtained was filtered to obtain a white powder of 3-(3-pyridyl)benzo[b]thiophen-6-ol (1.77g, 87%).
$^1$H-NMR(DMSO-$d_6$) δ: 6.94(dd, J=2.2, 8.8Hz, 1H), 7.37 (d, J=2.2Hz, 1H), 7.51(dd, J=4.9, 7.9Hz, 1H), 7.63(s, 1H), 7.65 (d, J=8.8Hz, 1H), 7.99(ddd, J=1.8, 2.1, 7.9Hz), 8.60(dd, J=1.5, 2.1, 7.9Hz, 1H), 8.78(d, J=1.8Hz, 1H), 9.78(s, 1H).
IR(KBr):3090-2600, 1594, 1468, 1249, 1049, 1038, 899, 837, 814, 774, 714cm$^{-1}$.
Melting point: 231.0-232.5°C

Example 9

Preparation of 3-(3-pyridyl)benzo[b]thiophen-6-yl=acetate

Acetic anhydride (0.1 ml, 1.060 mmol) was added to a suspension of 3-(3-pyridyl)benzo[b]thiophen-6-ol (100 mg, 0.4400 mmol) obtained in Example 8 in pyridine (5 ml), and the mixture was stirred for 3.5 hours at room temperature. After diluting the reaction mixture with ethyl acetate, the mixture was washed with water, saturated aqueous solution of sodium bicarbonate, and saturated brine, sequentially, and dried with anhydrous magnesium sulfate. The solvent was evaporated under reduced pressure. The residue was purified by crystallization in ethyl acetate-hexane to obtain a white powder of 3-(3-pyridyl)benzo[b]thiophen-6-yl=acetate (86 mg, 73%).
$^1$H-NMR($CDCl_3$) δ: 2.34 (s, 3H) , 7.14 (dd, J=2.1, 8.8Hz, 1H), 7.40(dd, J=4.9, 7.9Hz, 1H) 7.44(s, 1H), 7.66(d, J=2.1, 1H), 7.80(d, J=8.8Hz, 1H), 7.86(d, J=7.9, 1H), 8.64(d, J=3.3Hz, 1H), 8.81(s, 1H).
IR(KBr):2360, 1747, 1378, 1225, 1193, 814, 712cm$^{-1}$.
Melting point: 96.5-97.5°C.

Example 10

Preparation of 3-(3-pyridyl)benzo[b]thiophen-6-yl=benzoate

Benzoic anhydride (140 mg, 0.6188 mmol) was added to a suspension of 3-(3-pyridyl)benzo[b]thiophen-6-ol (100 mg, 0.4400 mmol) obtained in Example 8 in pyridine (5 ml), and the mixture was stirred for 24 hours at room temperature. After diluting the reaction mixture with diethyl ether, the mixture was washed with water, saturated aqueous solution of sodium bicarbonate, sequentially, and saturated brine and dried with anhydrous magnesium sulfate. The solvent was evaporated under reduced pressure. The resulting residue was submitted to silica gel column chromatography (hexane:ethyl acetate = 17:3). A white powder of 3-(3-pyridyl)benzo[b]thiophen-6-yl=benzoate (92 mg, 63%) was obtained from the eluate by crystallization in ethyl acetate-hexane.
$^1$H-NMR($CDCl_3$) δ: 7.28(dd, J=2.1, 8.8Hz, 1H), 7.42(ddd, J=0.9, 4.9, 7.9Hz, 1H), 7.47(s, 1H), 7.51-7.54(m, 2H), 7.63-7.66(m, 1H), 7.80 (d, J=2.1Hz, 1H), 7.85 (d, J=8.5Hz, 1H), 7.89 (ddd, J=1.8, 2.1, 7.9Hz, 1H), 8.22-8.24(m, 2H) , 8.66(dd, J=1.5, 4.9Hz, 1H), 8.85(d, J=1.5Hz, 1H).
IR(KBr):2365, 1731, 1264, 1201, 1087, 1070, 705cm$^{-1}$.
Melting point: 129.0-130.0 °C

Example 11

Preparation of 3-(6-benzyloxybenzo[b]thiophen-3-yl)pyridine hydrochloride

A suspension of the 3-(3-pyridyl)benzo[b]thiophen-6-ol (100 mg, 0.4400 mmol) obtained in Example 8, benzyl bromide (91 mg, 0.5297 mmol), and anhydrous potassium carbonate (80 mg, 0.5788 mmol) in dimethylformamide (DMF) (5ml) was stirred for 16 hours at 100°C. After cooling, the reaction mixture was diluted with ethyl acetate. The mixture was washed with water, then with saturated brine, and dried with anhydrous magnesium sulfate. The solvent was evaporated under reduced pressure. The resulting residue was submitted to silica gel column chromatography (hexane:ethyl acetate = 9:1) to obtain a colorless oily product of 3-(6-benzyloxybenzo[b]thiophen-3-yl)pyridine (60mg, 43%). The colorless oily product was dissolved indiethyl ether (3 ml). 1N hydrogen chloride-diethyl ether solution (1 ml, 1 mmol) was added to the product solution under ice cooling to obtain a precipitate. The precipitate was filtered to obtain a white powder of 3-(6-benzyloxybenzo[b]thiophen-3-yl)pyridine hydrochloride (45 mg, 29%).
$^1$H-NMR(DMSO-$d_6$) δ: 5.21(s, 2H), 7.18(dd, J=2.4, 8.8Hz, 1H), 7.33 (t, J=7.3Hz, 1H), 7.40(dd, J=7.0, 7.3Hz, 2H), 7.49 (d, J=7.0Hz, 2H), 7.80(d, J=2.1Hz, 1H), 7.82(d, J=9.1Hz, 1H), 7.97(s, 1H), 7.97(t, J=6.7Hz, 1H), 8.57(d, J=7.9Hz, 1H), 8.83(dd, J=1.5, 4.9Hz, 1H), 9.06(d, J=1.8Hz, 1H).
IR(KBr):3428, 2433, 2111, 1994, 1601, 1554, 1452, 1232cm$^{-1}$.
Melting point: 196.5-199.0°C

Example 12

Preparation of 3-(6-isopropyloxybenzo[b]thiophen-3-yl) pyridine hydrochloride

A suspension of 3-(3-pyridyl)benzo[b]thiophen-6-ol (100 mg, 0.4400 mmol) obtained in Example 8, isopropyl bromide (0.1 ml, 1.065 mmol), and anhydrous potassium carbonate (80 mg, 0.5788 mmol) in DMF (5ml) was stirred for 16 hours at 110°C. After cooling, the reaction mixture was diluted with diethyl ether. The mixture was washed with water, then with saturated brine, and dried with anhydrous magnesium sulfate. The solvent was evaporated under reduced pressure. The residue was submitted to silica gel column chromatography (hexane: ethyl acetate = 4:1) to obtain a colorless oily product of 3- (6-isopropyloxybenzo[b] thiophen-3-yl) pyridine (90 mg, 76%). The product was dissolved in diethyl ether (10 ml). 1N hydrogen chloride-diethyl ether solution (1 ml, 1 mmol) was added to the product solution under ice cooling to obtain a precipitate. The precipitate was filtered to obtain a white powder of 3-(6-isopropyloxybenzo[b]thiophen-3-yl)pyridine hydrochloride (74 mg, 55%).
$^1$H-NMR(DMSO-$d_6$) δ: 1.30(d, J=6.1Hz, 6H), 4.72(m, 1H), 7.07(dd, J=2.5, 8.8Hz, 1H), 7.70(d, J=2.5Hz, 1H), 7.80(d, J=8.8Hz, 1H), 7.99(s, 1H), 8.04(dd, J=5.5, 7.9Hz, 1H), 8.66(d, J=8.2Hz, 1H), 8.87(dd, J=1.2, 5.5Hz, 1H), 9.10(d, J=1.8Hz, 1H).
IR(KBr):3428, 2548, 2089, 1968, 1601, 1545, 1510, 1457, 1272, 1224, 1113, 1040, 971, 800cm$^{-1}$
Melting point: 152.5-154.0°C

Example 13

Preparation of 3-[6-(4-fluorophenyl)benzo[b]thiophen-3-yl] pyridine hydrochloride

$Tf_2O$ (0.97 ml, 5.766 mmol) was added to a suspension of the 3-(3-pyridyl)benzo[b]thiophen-6-ol (1.14 g, 5.016 mmol) obtained in Example 8 and 2,6-di-t-butyl-4-methylpyridine (1.25 g, 6.087 mmol) in dichloromethane (35 ml) under cooling with ice. The mixture was warmed to room temperature and stirred for 2.5 hours. The reaction mixture was concentrated under reduced pressure. The resulting residue was diluted with diethyl ether, washed with water, 5% citric acid aqueous solution, saturated aqueous solution of sodium bicarbonate, water, and then saturated brine, sequentially, and dried with anhydrous magnesium sulfate. The solvent was evaporated under reduced pressure. The resulting residue was submitted to basic silica gel column chromatography (hexane : ethyl acetate=93:7) to obtain a colorless oily product of 3-(3-pyridyl)benzo[b]thiophen-6-yl=trifluoromethane sulfonate (1.78 g, 99%).
$^1$H-NMR($CDCl_3$) δ: 7.32(dd, J=2.3, 8,9Hz, 1H), 7.42(dd, J=4.9, 7.9Hz, 1H), 7.57 (s, 1H), 7.84-7.86 (m, 3H), 8.67 (dd, J=2.1, 5.5Hz, 1H), 8.80(d, J=2.1Hz, 1H).

2M sodium carbonate aqueous solution (1.20 ml) was added to a solution of 3-(3-pyridyl)benzo[b]thiophen-6-yl= trifluoromethane sulfonate (200 mg, 0.5566 mmol), 4-fluorophenyl boronic acid (100 mg, 0.7147 mmol), and bistriphenylphosphine palladium (II) chloride (20.0mg, 0.02849 mmol) in THF (5 ml). The mixture was stirred for 22 hours at 80°C. The reaction mixture was cooled to room temperature and diluted with ether. Insoluble material was removed by filtration through Celite® (trademark, Wako Pure Chemical Industries, Ltd.). After removing the water layer from the filtrate, the filtrate was washed with water, then with saturated brine, and dried with anhydrous magnesium sulfate. The solvent was evaporated under reduced pressure. The residue obtained was submitted to silica gel column chromatography (hexane ethyl acetate = 19:1) to obtain a colorless oily product of 3-[6-(4-fluorophenyl)benzo[b]thiophen-3-yl]pyridine (151 mg, 89%). The product was dissolved in diethyl ether (5ml). 1N hydrogen chloride-diethyl ether solution (1 ml, 1 mmol) was added to the product solution under ice cooling to obtain a precipitate. The precipitate was filtered to obtain a white powder of 3-[6-(4-fluorophenyl)benzo[b]thiophen-3-yl]pyridine hydrochloride (144 mg, 76%).
$^1$H-NMR(DMSO-$d_6$) δ: 7.31-7.36(m, 2H), 7.79(dd, J=1.8, 8.5Hz, 1H), 7.81-7.84(m, 2H), 8.00(d, J=7.9Hz, 1H), 8.22(s, 1H), 8.45(d, J=1.2Hz, 1H), 8.65(d, J=8.2Hz, 1H), 8.87(dd, J=1.2, 5.3Hz, 1H), 9.13(d, J=1.8Hz, 1H).
IR(KBr):2360, 2112, 1993, 1564, 1517, 1505, 1320, 1233, 828, 810$cm^{-1}$.
Melting point: 232.0-235.0°C

Example 14

Preparation of 3-[6-(3-fluorophenyl)benzo[b]thiophen-3-yl] pyridine hydrochloride

2M sodium carbonate aqueous solution (1.20 ml) was added to a solution of 3-(3-pyridyl)benzo[b]thiophen-6-yl= trifluoromethane sulfonate (200 mg, 0.5566 mmol) obtained in Example 13, 3-fluorophenyl boronic acid (100 mg, 0.7147 mmol), and bistriphenylphosphine palladium (II) chloride (20.0 mg, 0.02849 mmol) in THF (5 ml). The mixture was stirred for 22 hours at 80°C. The reaction mixture was cooled to room temperature and diluted with diethyl ether. Insoluble material was removed by filtration through Celite® (Wako Pure Chemical Industries, Ltd.). After removing the water layer from the filtrate, the filtrate was washed with water, then with saturated brine, and dried with anhydrous magnesium sulfate. The solvent was evaporated under reduced pressure. The resulting residue was submitted to silica gel column chromatography (hexane:ethyl acetate = 19:1) to obtain a colorless oily product of 3-[6-(4-fluorophenyl) benzo[b]thiophen-3-yl]pyridine (155 mg, 91%). The product was dissolved in diethyl ether (5 ml). 1N hydrogen chloride-diethyl ether solution (1 ml, 1 mmol) was added to the product solution under ice cooling to obtain a precipitate. The precipitate was filtered to obtain a pale yellow powder of 3-[6-(4-fluorophenyl)benzo[b]thiophen-3-yl]pyridine hydrochloride (150 mg, 79%).
$^1$H-NMR(DMSO-$d_6$) δ: 7.21-7.24(m, 1H), 7.52-7.56(m, 1H), 7.64-7.66(m, 2H), 7.85(dd, J=1.5, 8.6Hz, 1H), 8.00(d, J=8.2Hz, 1H), 8.02(dd, J=5.5, 7.9Hz, 1H), 8.53(d, J=1.5Hz, 1H), 8.64(d, J=7.9Hz, 1H), 8.88(dd, J=1.2, 5.5Hz, 1H), 9.12(d, J=1.8Hz, 1H). IR(KBr):3429, 2359, 2111, 1996, 1583, 1565, 1547, 1320, 1263, 1182, 820, 800, 782, 695, 625$cm^{-1}$.
Melting point: 217.5-220.5°C

Example 15

Preparation of 3-(6-phenylbenzo[b]thiophen-3-yl)pyridine

2M sodium carbonate aqueous solution (1.20 ml) was added to a solution of 3-(3-pyridyl)benzo[b]thiophen-6-yl= trifluoromethane sulfonate (200 mg, 0.5566 mmol) obtained in Example 13, phenyl boronic acid (90 mg, 0.7381 mmol), and bistriphenylphosphine palladium (II) chloride (20.0mg, 0.02849 mmol) in THF (5 ml). The mixture was stirred for 4 hours at 80°C. The reaction mixture was cooled to room temperature and diluted with diethyl ether. Insoluble material was removed by filtration through Celite® (Wako Pure Chemical Industries, Ltd.). After removing the water layer from the filtrate, the filtrate was washed with water, then with saturated brine, and dried with anhydrous magnesium sulfate. The solvent was evaporated under reduced pressure. The residue obtained was submitted to basic silica gel column chromatography (hexane:ethyl acetate = 19:1). A white powder of 3-(6-phenylbenzo[b]thiophen-3-yl)pyridine (119 mg, 74%) was obtained from the eluate by crystallizing in ethyl acetate-hexane.
$^1$H-NMR($CDCl_3$) δ: 7.37(dd, J=7.3, 7.6Hz, 1H), 7.42(dd, J=4.9, 7.6Hz, 1H) , 7.47 (t, J=7.9Hz, 2H) , 7.48(s, 1H), 7.64-7.67 (m, 3H), 7.90(d, J=8.5Hz, 1H), 7.92(ddd, J=1.8, 2.1, 7.9Hz, 1H), 8.13(d, J=1.5Hz, 1H), 8.66(dd, J=1.5, 4.9Hz, 1H), 8.87(d, J=2.1Hz 1H).
IR(KBr) :2361, 1771, 1456, 1420, 1320, 1186, 1026, 816, 778, 745, 716,706cm$^{-1}$.
Melting point: 131.5-132.5°C

Example 16

Preparation of 3-[6-(3-methoxyphenyl]benzo[b]thiophen-3-yl] pyridine hydrochloride

2M sodium carbonate aqueous solution (1.80 ml) was added to a solution of 3-(3-pyridyl)benzo[b]thiophen-6-yl-trifluoromethane sulfonate (300 mg, 0.8348 mmol) obtained in Example 13, 3-methoxyphenyl boronic acid (140 mg, 0.9213 mmol), and bistriphenylphosphine palladium (II) chloride (30.0 mg, 0.04274 mmol) in THF (8 ml). The mixture was stirred for 4 hours at 80°C. The reaction mixture was cooled to room temperature and diluted with diethyl ether. Insoluble material was removed by filtration through Celite® (Wako Pure Chemical Industries, Ltd.). After removing the water layer from the filtrate, the filtrate was washed with water, then with saturated brine, and dried with anhydrous magnesium sulfate. The solvent was evaporated under reduced pressure. The resulting residue was submitted to silica gel column chromatography (hexane:ethyl acetate = 93:7) to obtain a colorless oily product of 3-[6-(3-methoxyphenyl)benzo[b] thiophen-3-yl]pyridine (249 mg, 94%). The product was dissolved in diethyl ether (5ml). 1N hydrogen chloride-diethyl ether solution (1 ml, 1 mmol) was added to the product solution under ice cooling to obtain a precipitate. The precipitate was filtered to obtain a white powder of 3-[6-(3-methoxyphenyl)benzo[b]thiophen-3-yl]pyridine hydrochloride (225 mg, 76%).
$^1$H-NMR(DMSO-$d_6$) δ: 3.84(s, 3H), 6.97(ddd, J=0.9, 2.4, 7.9Hz, 1H), 7.31(dd, J=1.8, 2.4Hz, 1H), 7.35(d, J=8.3Hz, 1H), 7.41(t, J=7.9Hz, 1H), 7.81(dd, J=1.5, 8.5Hz, 1H), 7.99(d, J=8.5Hz, 1H), 8.07(dd, 5.5, 7.9Hz, 1H), 8.48(d, J=1.2Hz, 1H), 8.71 (ddd, J=1.5, 1.8, 8.2Hz, 1H), 8.90(dd, J=1.2, 5.5Hz, 1H), 9.15(d, J=1.8Hz, 1H).
IR(KBr):3411, 2830, 2356, 2078, 1957, 1608, 1580, 1558, 1466, 1282, 1212, 1171, 830, 800, 777, 768, 692, 624cm$^{-1}$.
Melting point: 162.5-164.5°C

Example 17

Preparation of 3-[3-(3-pyridyl)benzo[b]thiophen-6-yl]-phenol

47% hydrobromide solution (5 ml) was added to the 3-[6-(3-methoxyphenyl)benzo[b]thiophen-3-yl]pyridine (87 mg, 0.2741mmol) obtained in Example 16, and the mixture was refluxed for 1.5 hours. After neutralizing with 5N sodium hydroxide aqueous solution and saturated aqueous solution of sodium bicarbonate, the reaction mixture was extractedwithmethyl ethyl ketone. The organic layer was washed with water, then with saturated brine, and dried with anhydrous magnesium sulfate. The solvent was evaporated under reduced pressure. The resulting residue was purified by crystallizing in ethyl acetate-hexane to obtain a white powder of 3-[3-(3-pyridyl) benzo[b]thiophen-6-yl]phenol (65 mg, 78%).
$^1$H-NMR(DMSO-$d_6$) δ:6.79(dd, J=1.6, 8.0Hz, 1H), 7.12(dd, J=1.8, 2.1Hz, 1H), 7.17 (d, J=7.9Hz, 1H), 7.27 (t, J=7.9Hz, 1H), 7.56(dd, J=4.7, 7.9Hz, 1H), 7.69(dd, J=1.8, 8.5Hz, 1H), 7.92(d, J=8.2Hz, 1H), 7.99(s, 1H), 8.06(ddd, J=1.8, 2.1, 7.9Hz, 1H), 8.34(d, J=1.5Hz, 1H), 8.64(dd, J=1.5, 4.9Hz, 1H), 8.85(d, J=1.8Hz, 1H), 9.55(s, 1H).
IR(KBr):3049, 1580, 1467, 1448, 1418, 1307, 1293, 1198, 813, 795, 775, 711, 701cm$^{-1}$.
Melting point: 188.5-189.5°C
Mass:304(M+H)

Example 18

Preparation of 3-[6-(4-methoxyphenyl)benzo[b]thiophen-3-yl] pyridine

2M sodium carbonate aqueous solution (1.20 ml) was added to a solution of 3-(3-pyridyl)benzo[b]thiophen-6-yl= trifluoromethane sulfonate (200 mg, 0.5566 mmol) obtained in Example 13, 4-methoxyphenyl boronic acid (100 mg, 0.6581 mmol), and bistriphenylphosphine palladium (II) chloride (20.0 mg, 0.02849 mmol) in THF (5 ml). The mixture was stirred for 4 hours at 80°C. The reaction mixture was cooled to room temperature and diluted with diethyl ether. Insoluble material was removed by filtration through Celite® (Wako Pure Chemical Industries, Ltd.). After removing the water layer from the filtrate, the filtrate was washed with water, then with saturated brine, and dried with anhydrous magnesium sulfate. The solvent was evaporated under reduced pressure. The residue was submitted to basic silica gel column chromatography (hexane:ethyl acetate = 93:7). A white powder of 3-[6-(4-methoxyphenyl)benzo [b] thiophen-3-yl]pyridine (141mg, 80%) was obtained from the eluate by crystallizing in ethyl acetate-hexane.
$^1$H-NMR($CDCl_3$) δ: 3.85(s, 3H), 7.00(d, J=8.5Hz, 2H), 7.42(dd, J=4.7, 7.8Hz, 1H), 7.45(s,1H), 7.59-7.62(m,3H), 7.88 (d,J=8.2Hz, 1H), 7.91(ddd, J=1.8, 2.1, 7.9Hz, 1H), 8.08(d, J=1.5Hz, 1H), 8.65(dd, J=1.6, 4.8Hz, 1H), 8.86(d, J=2.4Hz, 1H).
IR(KBr) :2362, 1510, 1247, 1185, 1035, 1020, 813, 718, 669cm$^{-1}$.
Melting point: 122.5-123.0°C

Example 19

Preparation of 4-[3-(3-pyridyl)benzo[b]thiophen-6-yl]-phenol

47% hydrobromide solution (4 ml) was added to the 3-[6-(4-methoxyphenyl)benzo[b]thiophen-3-yl]pyridine (92 mg, 0.2898 mmol) obtained in Example 18, and the mixture was refluxed for 1 hour. The reaction mixture was neutralized with 5N sodium hydroxide aqueous solution and saturated sodium bicarbonate solution to form crystals. The crystals were filtered to obtain a white powder of 4-[3-(3-pyridyl)benzo[b]thiophen-6-yl]phenol (91 mg).
$^1$H-NMR(DMSO-$d_6$) δ: 6.88(d, 8.5Hz, 2H), 7.60(d, J=8.5Hz, 2H), 7.70(dd, J=1.8, 8.5Hz, 1H), 7.76(dd, J=5.2, 7.9Hz, 1H), 7.90 (d, J=8.5Hz, 1H), 8.03(s, 1H), 8.32(m, 2H), 8.74(d, J=4.3Hz, 1H), 8.98(br s, 1H), 9.60(br s, 1H).
IR(KBr):3057, 1609, 1508, 1451, 1268, 1177, 815cm$^{-1}$.
Melting point: 181.5-184.5°C

Example 20

Preparation of 3-[6-(3-pyridyl)benzo[b]thiophen-3-yl] pyridine

2M sodium carbonate aqueous solution (0.90 ml) was added to a solution of 3-(3-pyridyl)benzo[b]thiophen-6-yl= trifluoromethane sulfonate (150 mg, 0.4174 mmol) obtained in Example 13, diethyl (3-pyridyl)borane (73 mg, 0.4965 mmol), and bistriphenylphosphine palladium (II) chloride (20.0mg, 0.03134 mmol) in THF (4 ml). The mixture was stirred for 4 hours at 80°C. The reaction mixture was cooled to room temperature and diluted with diethyl ether. Insoluble material was removed by filtration through Celite® (Wako Pure Chemical Industries, Ltd.). After removing the water layer from the filtrate, the filtrate was washed with water and extracted with 1N hydrochloric acid. The water layer was adjusted to pH 8 with 5N sodium hydroxide and was extracted with diethyl ether. The organic layer was washed with water, then with saturated brine, and dried with anhydrous magnesium sulfate. The solvent was evaporated under reduced pressure. The residue obtained was crystallized in diethyl ether-hexane to obtain a white powder of 3-[6-(3-pyridyl)benzo[b]thiophen-3-yl]pyridine (50 mg, 42%).
$^1$H-NMR($CDCl_3$) δ: 7.39(ddd, J=0.6, 4.8, 7.9Hz, 1H), 7.43(ddd, J=0.6, 4.9, 7.9Hz, 1H), 7.52 (s, 1H), 7.62(dd, J=1.6, 8.4Hz, 1H), 7.90-7.96(m, 2H), 7.93(d, J=8.0Hz, 1H), 8.12(d, J=1.2Hz, 1H), 8.61(dd, J=1.3, 4.8Hz, 1H), 8.66(dd, J=1.5, 4.6Hz, 1H), 8.86(d, J=1.5Hz, 1H), 8.92(d, J=1.8Hz, 1H).
IR(KBr):3419, 1576, 1479, 1458, 1414, 1325, 797, 713cm$^{-1}$.
Melting point: 131.5-133.0°C Mass: 289 (M+H)

Example 21

Preparation of 3-[6-(3,4-dimethoxyphenyl)benzo[b]thiophen-3-yl]pyridine

2M sodium carbonate aqueous solution (1.80 ml) was added to a solution of 3-(3-pyridyl)benzo[b]thiophen-6-yl= trifluoromethane sulfonate (300 mg, 0.8348 mmol) obtained in Example 13, 3,4-dimethoxyphenyl boronic acid

(168 mg, 0.9238 mmol), and bistriphenylphosphine palladium (II) chloride (30.0 mg, 0.04274 mmol) in THF (6ml). The mixture was stirred for 3 hours at 80°C. The reaction mixture was cooled to room temperature and diluted with diethyl ether. Insoluble material was removed by filtration through Celite® (Wako Pure Chemical Industries, Ltd.). After removing the water layer from the filtrate, the filtrate was washed with water, then with saturated brine, and dried with anhydrous magnesium sulfate. The solvent was evaporated under reduced pressure. The resulting residue was submitted to basic silica gel column chromatography (diethyl ether:hexane = 9:1). A white powder of 3-[6-(3,4-dimethoxyphenyl)benzo[b]thiophen-3-yl]pyridine (195 mg, 67%) was obtained from the eluate by crystallizing in diethyl ether-hexane.
$^1$H-NMR($CDCl_3$) δ: 3.93(s, 3H), 3.97(s, 3H), 6.97 (d, J=8.5Hz, 1H), 7.17(d, J=2.1Hz, 1H), 7.21(dd, J=2.1, 8.2Hz, 1H), 7.42(ddd, J=0.9, 4.9, 7.9Hz, 1H), 7.46 (s, 1H), 7.61(dd, J=1.6, 8.4Hz, 1H), 7.87(d, J=8.2Hz, 1H), 7.91(ddd, J=1.6, 2.3, 7.6Hz, 1H), 8.08(d, J=1.6Hz, 1H), 8.65(dd, J=1.6, 4.9Hz, 1H), 8.86(d, J=1.6Hz, 1H).
IR(KBr):2830, 1516, 1457, 1435, 1273, 1253, 1175, 1149, 1136, 1024, 811,788, 771, 713cm$^{-1}$.
Melting point: 102.5-105.0°C

Example 22

Preparation of 4-[3-(3-pyridyl)benzo[b]thiophen-6-yl]benzene -1,2-diol

47% hydrobromide solution (10 ml) was added to the 3-[6-(3,4-dimethoxyphenyl)benzo[b]thiophen-3-yl]pyridine (150 mg, 0.4317 mmol) obtained in Example 21, and the mixture was refluxed for 1 hour. The reaction mixture was neutralized with 5N sodium hydroxide aqueous solution and saturated sodium bicarbonate aqueous solution to form crystals. The crystals formed were filtered and recrystallized in ethyl acetate- ethanol-hexane to obtain a white powder of 4-[3-(3-pyridyl)benzo [b]thiophen-6-yl]benzene-1,2-diol (133 mg, 96%).
$^1$H-NMR(DMSO-$d_6$) δ: 6.84(d, 8.2Hz, 1H), 7.04(dd, J=2.1, 7.9Hz, 1H), 7.13(d, J=2.1Hz, 1H), 7.56(dd, J=4.7, 7.9Hz, 1H), 7.63(dd, J=1.5, 8.5Hz, 1H), 7.87(d, J=8.5Hz, 1H), 7.94(s, 1H), 8.06(d, J=7.9Hz, 1H), 8.23(d, J=1.2Hz, 1H), 8.63 (dd, J=1.5, 4.8Hz, 1H), 8.84(d, J=1.8Hz, 1H), 9.08(br s, 2H).
IR(KBr) :3450, 1599, 1507, 1442, 1424, 1293, 1270, 1191, 1118, 812, 778, 711cm$^{-1}$.
Melting point: 235.5-237.5 °C

Example 23

Preparation of 3-[3-(3-pyridyl)benzo[b]thiophen-6-yl] phenylamine

2M sodium carbonate aqueous solution (1.20 ml) was added to a solution of 3-(3-pyridyl)benzo[b]thiophen-6-yl-trifluoromethane sulfonate (190 mg, 0.5287 mmol) obtained in Example 13, 3-aminophenyl boronic acid (97 mg, 0.6260 mmol), and bistriphenylphosphine palladium (II) chloride (28.0 mg, 0.03989 mmol) in THF (5 ml). The mixture was stirred for 2.5 hours at 80°C. The reaction mixture was cooled to room temperature and diluted with diethyl ether. Insoluble material was removed by filtration through Celite® (Wako Pure Chemical Industries, Ltd.). After removing the water layer from the filtrate, the filtrate was washed with water, and extracted with 1N hydrochloric acid. The water layer was adjusted to pH 8 with 5N sodium hydroxide and extracted with diethyl ether. The organic layer was washed with water, then with saturated brine, and dried with anhydrous magnesium sulfate. The solvent was evaporated under reduced pressure. The residue was purified by crystallizing in ethyl acetate-hexane to obtain a white powder of 3- [3-(3-pyridyl)benzo [b] thiophen-6-yl]phenylamine (114 mg, 71%).
$^1$H-NMR(DMSO-$d_6$) δ: 5.17(br s, 2H), 6.58(dd, J=1.8, 7.9Hz, 1H), 6.88 (d, J=7.6Hz, 1H), 6.94 (t, J=1.8Hz, 1H), 7.12 (dd, J=7.6, 7.9Hz, 1H), 7.56(dd, J=4.9, 7.6Hz, 1H), 7.66(dd, J=1.8, 8.5Hz, 1H), 7.91(d, J=8.5Hz, 1H), 7.98(s, 1H), 8.08 (ddd, J=1.8, 2.1, 7.9Hz, 1H), 8.27(d, J=1.5Hz, 1H), 8.64(dd, J=1.5, 4.9Hz, 1H), 8.85(d, J=2.1Hz, 1H).
IR(KBr):3375, 2358, 1605, 1469, 1418, 794, 770, 715, 699cm$^{-1}$.
Melting point: 184.0-185.5°C

Example 24

Preparation of 3-[3-(3-pyridyl)benzo[b]thiophen-6-yl] acetylaminobenzene

Acetic anhydride (0.1ml, 1.06mmol) was added to a solution of 3-[3-(3-pyridyl)benzo[b] thiophen-6-yl]phenylamine (70 mg, 0.2315 mmol) obtained in Example 23 in pyridine (3 ml) under ice cooling, and the mixture was stirred for 2 hours while increasing the temperature to room temperature. After diluting the reaction mixture with diethyl ether, the mixture was washed with water, saturated aqueous solution of sodium bicarbonate, and saturated brine, sequentially, and dried with anhydrous magnesium sulfate. The solvent was evaporated under reduced pressure. The resulting

residue was crystallized in ethyl-acetate-diethyl ether-hexane to obtain a white powder of 3-[3-(3-pyridyl) benzo[b] thiophen-6-yl]acetylaminobenzene (57 mg, 71%).
$^1$H-NMR($CDCl_3$) $\delta$: 2.20 (s, 3H), 7.35-7.47(m, 6H), 7.62(dd, J=1.8, 8.4Hz,1H), 7.86-7.88(m, 2H), 7.90(ddd, J=1.8, 2.1, 8.0Hz, 1H), 8.12(d, J=1.5Hz,1H), 8.65(dd, J=1.5, 4.9Hz,1H), 8.86(d, J=1.8Hz, 1H).
IR(KBr):3265, 1668, 1610, 1589, 1554, 1489, 1418, 1318, 785, 713cm$^{-1}$.
Melting point: 92.0-96.5°C (decomposed)

Example 25

Preparation of 3-(6-cyclopentyloxybenzo[b]thiophen-3-yl) pyridine hydrochloride

A suspension of 3-(3-pyridyl)benzo[b]thiophen-6-ol (100 mg, 0.4400 mmol) obtained in Example 8, cyclopentyl bromide (79.0 mg, 0.5301 mmol), and anhydrous potassium carbonate (80 mg, 0.5788 mmol) in DMF (5 ml) was stirred for 21.5 hours at 75°C. After cooling, the reaction mixture was diluted with diethyl ether. The mixture was washed with water, then with saturated brine, and dried with anhydrous magnesium sulfate. The solvent was evaporated under reduced pressure. The residue obtained was submitted to basic silica gel column chromatography (hexane: ethyl acetate = 17:3) to obtain 3-(6-cyclopentyloxybenzo[b] thiophen-3-yl)pyridine (95 mg, 73%). This product was dissolved in diethyl ether. 1N hydrogen chloride-diethyl ether solution was added to the product solution under ice cooling to obtain a precipitate. The precipitate was filtered to obtain a powder of 3-(6-cyclopentyloxybenzo[b]thiophen-3-yl)pyridine hydrochloride (88 mg, 60%).
$^1$H-NMR(DMSO-$d_6$) $\delta$: 1.60 (m, 2H), 1.72 (m, 4H), 1.96 (m, 2H), 4.92 (m, 1H), 7.06 (dd, J=2.4, 8.9Hz, 1H), 7.66 (d, J=2.4Hz, 1H), 7.78 (d, J=8.9Hz, 1H), 7.97 (s, 1H), 8.01 (dd, J=5.5, 7.9Hz, 1H), 8.62 (dt, J=1.5, 8.2Hz, 1H), 8.85 (dd, J=1.3, 5.5Hz, 1H), 9.08 (d, J=1.8Hz, 1H).
IR (KBr): 3010, 2957, 1600, 1543, 1499, 1458, 1315, 1230, 1176, 1041, 988, 835, 918, 689, 618 cm$^{-1}$.

Example 26

Preparation of 3-[6-(3-methoxyphenoxy)benzo[b]thiophen-3-yl] pyridine hydrochloride

Triethylamine (0.3 ml, 2.152 mmol) was added to a suspension of 3-(3-pyridyl)benzo[b]thiophen-6-ol (100 mg, 0.4400 mmol) obtained in Example 8,3-methoxyphenyl boronic acid (140 mg, 0.9213 mmol), copper acetate (80.0 mg, 0.4404 mmol), and a small amount of powder molecular sieve 4Å in methylene chloride (4.5 ml). The mixture was stirred for 4 days at room temperature. Insoluble material was removed by filtration through Celite® (trademark, Wako Pure Chemical Industries, Ltd.) and the filtrate was concentrated under reduced pressure. The residue was submitted to silica gel column chromatography (hexane:ethyl acetate = 17:3) to obtain 3-[6-(3-methoxyphenoxy)benzo[b] thiophen-3-yl]pyridine (40mg, 27%). This product was dissolved in ethanol. 1N hydrogen chloride-diethyl ether solution was added to the product solution under ice cooling to obtain a precipitate. The precipitate was filtered to obtain a powder of 3- [6-(3-methoxyphenoxy)benzo[b]thiophen-3-yl] pyridine hydrochloride (39 mg, 24%).
$^1$H-NMR(DMSO-$d_6$) $\delta$: 3.73 (s, 3H), 6.57 (m, 1H), 6.63 (dd, J=2.1, 2.5Hz, 1H), 6.73 (dd, J=2.5, 8.2Hz, 1H), 7.21 (dd, J=2.1, 8.5Hz, 1H), 7.29 (t, J=8.2Hz, 1H), 7.81 (d, J=2.1Hz, 1H), 7.94 (d, J=8.9Hz, 1H), 7.97 (dd, J=5.2, 7.9Hz, 1H) , 8.09 (s, 1H) , 8.57 (brd, J=7.9Hz, 1H), 8.84 (dd, J=1.5, 5.5Hz, 1H), 9.08 (d, J=1.8Hz, 1H).
IR (KBr) : 3423, 3059, 2506, 2059, 1590, 1556, 1488, 1283, 1220, 1143, 1035, 964, 790, 685 cm$^{-1}$.

Example 27

Preparation of 3-(6-methylbenzo[b]thiophen-3-yl)pyridine hydrochloride

3-methylbenzenethiol (1.0 ml, 8.405 mmol) was added to a solution of potassium hydroxide (1.29g, 19.54mmol) in a mixture of water (5ml) and ethanol (7ml) under cooling with ice. After the further addition of 2-bromo-1-pyridin-3-yl ethanone hydrobromide (2.83 g, 10.07 mmol), the mixture was stirred for 2.5 hours at room temperature. After removing the ethanol by vacuum evaporation, the residue was extracted with diethyl ether. The organic layer was washed with water, then with saturated brine, dried with anhydrous magnesium sulfate, then the solvent was evaporated under reduced pressure. The resulting residue was charged to silica gel column chromatography (hexane:ethyl acetate = 3:2) to obtain crude 2-(3-methylphenylsulfanyl)-1-pyridin-3-yl ethanone (1.63 g, 80%).
$^1$H-NMR($CDCl_3$) $\delta$: 2.31(s, 3H), 4.22(s, 2H), 7.06(m, 1H), 7.18(m, 3H), 7.42(ddd, J=0.6, 4.9, 8.0Hz, 1H), 8.20(ddd, J=1.9, 2.1, 8.0Hz, 1H), 8.78(dd, J=1.5, 4.9Hz, 1H), 9.13(d, J=1.5Hz, 1H).
The crude 2-(3-methylphenylsulfanyl)-1-pyridin-3-yl ethanone (0.48 g, 1.973 mmol) obtained in the above procedure was dissolved in 1,2-dichloroethane (10 ml). This solution was dissolved in borontrifluoride-diethyl ether

complex (0.5 ml, 3.946mol). The solution was refluxed for 15 hours under nitrogen stream while heating. The reaction solution was cooled, and water and 5N sodium hydroxide aqueous solution were added under cooling with ice, to obtain a pH 8 water layer. The product was extracted with diethyl ether. The organic layer was washed with water, then with saturated brine and dried with anhydrous magnesium sulfate. The solvent was evaporated under reduced pressure and the resulting residue was charged to silica gel column chromatography (hexane:ethyl acetate = 17:3) to obtain 3-[6- methylbenzo[b]thiophen-3-yl]pyridine (170 mg, 38%). This product was dissolved in ethanol. 1N hydrogen chloride-diethyl ether solution was added to the product solution under ice cooling to obtain a precipitate. The precipitate was filtered to obtain a powder of 3-(6-methylbenzo[b]thiophen-3-yl)pyridine hydrochloride (146 mg, 28%).
$^1$H-NMR(DMSO-$d_6$) δ: 2.46(s, 3H), 7.32(d, J=7.9Hz, 1H), 7.83(d, J=8.2Hz, 1H), 7.92(br s, 1H), 8.05(dd, J=5.5, 7.9Hz, 1H), 8.12(s, 1H), 8.68(d, J=7.9Hz, 1H), 8.88 (d, J=4.9Hz, 1H), 9.11 (br s, 1H).

Example 28

Preparation of 3-(6-bromobenzo[b]thiophen-3-yl)pyridine hydrochloride

3-bromobenzenethiol (1.0 ml, 9.689 mmol) was added to a solution of potassium hydroxide (1.41g, 21.36 mmol) in a mixture of water (5.5 ml) and ethanol (7.7 ml) under cooling with ice. After the further addition of 2-bromo-1-pyridin-2-yl ethanone hydrobromide (2.99 g, 10.64 mmol), the mixture was stirred for 3 hours at room temperature. After removing ethanol by vacuum evaporation, the residue was extracted with diethyl ether. The organic layer was washed with water, then with saturated brine, dried with anhydrous magnesium sulfate, and the solvent was evaporated under reduced pressure. The resulting residue was recrystallized in chloroform-ethyl acetate-hexane to obtain colorless crystals of 2-(3-bromophenylsulfanyl)-1-pyridin -3-yl ethanone (1.18 g, 56%).
$^1$H-NMR($CDCl_3$) δ: 4.24 (s, 2H), 7.14(t, J=8.0H 1H), 7.28(m, 1H), 7.35(m, 1H), 7.41(dd, J=4.9, 8.0Hz, 1H), 7.50(t, J=1.8Hz, 1H), 8.19(ddd, J=1.8, 2.1, 8.0Hz, 1H), 8.78(dd, J=1.8, 4.9Hz, 1H), 9.13(d, J=1.5Hz, 1H).

Polyphosphoric acid (3.0 g) was stirred at 90°C with heating and the 2-(3-bromophenylsulfanyl)-1-pyridin-3-yl ethanone (0.31 g, 1.006 mmol) obtained above was added little by little thereto. The mixture was stirred for 3 hours at 90°C. The reacted solution was cooled and, after the addition of ice water, 5N sodium hydroxide aqueous solution was added to obtain a pH 8 water layer. The product was extracted with diethyl ether. The organic layer was washed with water, then with saturated brine and dried with anhydrous magnesium sulfate. The solvent was evaporated under reduced pressure and the residue obtained was charged to silica gel column chromatography (hexane:ethyl acetate = 17:3) to obtain 3-[6-bromobenzo[b]thiophen-3-yl] pyridine (43 mg, 15%). This product was dissolved in diethyl ether. 1N hydrogen chloride-diethyl ether solution was added to the product solution under ice cooling to obtain a precipitate . The precipitate was filtered to obtain a powder of 3-(6-bromobenzo[b]thiophen-3-yl)pyridinehydrochloride(49mg, 15%).
$^1$H-NMR(DMSO-$d_6$) δ: 7.63(dd, J=1.8, 8.9Hz, 1H), 7.85(d, J=8.9Hz, 1H), 7.96(dd, J=5.5, 7.9Hz, 1H), 8.18(s, 1H), 8.45 (d, J=1.5Hz, 1H), 8.54(d, J=7.9Hz, 1H), 8.84(d, J=5.2Hz, 1H), 9.06(d, J=1.5Hz, 1H).

Example 29

Preparation of 3-(4-bromobenzo[b]thiophen-3-yl)pyridine hydrochloride

Polyphosphoric acid (3.0 g) was stirred at 90°C with heating and the 2-(3-bromophenylsulfanyl)-1-pyridin-3-yl ethanone (0.31 g, 1.006 mmol) obtained in Example 28 was added little by little thereto. The mixture was stirred for 3 hours at 90°C. The reacted solution was cooled. After the addition of ice water, 5N sodium hydroxide aqueous solution was added to the mixture to obtain a pH 8 water layer. The product was extracted with diethyl ether. The organic layer was washed with water, then with saturated brine and dried with anhydrous magnesium sulfate. The solvent was evaporated under reduced pressure and the resulting residue was charged to silica gel column chromatography (hexane:ethyl acetate = 17:3) to obtain 3-[4-bromobenzo[b]thiophen-3-yl]pyridine (110 mg, 38%). This product was dissolved in diethyl ether. 1N hydrogen chloride-diethyl ether solution was added to the product solution under ice cooling to obtain a precipitate. The precipitate was filtered to obtain a powder of 3- (4-bromobenzo[b]thiophen-3-yl) pyridine hydrochloride (118 mg, 36%).
$^1$H-NMR(DMSO-$d_6$) δ: 7.38(dd, J=7.6, 8.2Hz, 1H), 7.68(d, J=7.6Hz, 1H), 8.06(dd, J=5.5, 8.0Hz, 1H), 8.13(s, 1H) , 8.21 (d, J=8.2Hz, 1H), 8.58(d, J=7.9Hz, 1H), 8.94(d, J=4.8Hz, 1H), 9.07(br s, 1H).

Example 30

Preparation of 3-(6-cyclobutyloxybenzo[b]thiophen-3-yl) pyridine hydrochloride

A suspension of 3-(3-pyridyl)benzo[b]thiophen-6-ol (100 mg, 0.4400 mmol) obtained in Example 8, cyclobutyl

bromide (0.05 ml, 0.5296 mmol), and anhydrous potassium carbonate (80 mg, 0.5788 mmol) in DMF (5 ml) was stirred for 16 hours at 75°C. The reaction mixture was cooled. After the addition of cyclobutyl bromide (0.05 ml, 0.5296 mmol) and anhydrous potassium carbonate (80 mg, 0.5788 mmol), the mixture was stirred for 23 hours at 75°C. After cooling, the reaction mixture was diluted with diethyl ethyl. The mixture was washed with water, then with saturated brine, and dried with anhydrous magnesium sulfate. The solvent was evaporated under reduced pressure. 1N hydrogen chloride-diethyl ether solution was added to the residue to obtain a precipitate, which was then filtered. The filtered precipitate was dissolved in ethanol. Diethyl ether was added to the solution to obtain a precipitate. The precipitate was filtered to obtain a powder of 3-(6-cyclobutyloxybenzo[b] thiophen-3-yl)pyridine hydrochloride (69 mg, 49%).
$^1$H-NMR(DMSO-$d_6$) δ: 1.67 (m, 1H), 1.80 (m, 1H), 2.08 (m, 2H), 4.79 (m, 1H), 7.05 (d, J=8.6Hz, 1H), 7.57 (br s, 1H), 7.81 (d, J=8.8Hz, 1H), 8.00(s, 1H), 8.05 (dd, J=5.8, 7.6Hz, 1H), 8.66 (d, J=7.0Hz, 1H), 8.88 (d, J=5.5Hz, 1H), 9.11 (s, 1H).
IR (KBr): 3402, 3220, 3043, 2940, 2344, 2089, 1599, 1550, 1509, 1456, 1272, 1227, 1082, 823, 805, 684cm$^{-1}$.

Example 31

Preparation of 3-[6-(3-thienyl)benzo[b]thiophen-3-yl] pyridine hydrochloride

2M sodium carbonate aqueous solution (0.45 ml) was added to a solution of 3-(3-pyridyl)benzo[b]thiophen-6-yl-trifluoromethane sulfonate (105 mg, 0.2922 mmol) obtained in Example 13, thiophene-3-boronic acid (50.0 mg, 0.3907 mmol), and bistriphenylphosphine palladium (II) chloride (10.0 mg, 0.01425 mmol) in THF (4 ml). The mixture was stirred for 3 hours at 80°C. The reaction mixture was cooled and diluted with ethyl acetate. Insoluble material was removed by filtration through Celite® (trademark, Wako Pure Chemical Industries, Ltd.). After removing the water layer from the filtrate, the filtrate was washed with water, then with saturated brine, and extracted with 6N hydrochloric acid. The extract was alkalinized with the addition of 50% sodium hydroxide aqueous solution, and extracted with ethyl acetate. The organic layer was washed with water, then with saturated brine, and dried with anhydrous magnesium sulfate. The solvent was evaporated under reduced pressure. The resulting residue was dissolved in diethyl ether. 1N hydrogen chloride-diethyl ether solution was added to the dissolved solution to obtain a precipitate. The precipitate was filtered to obtain a powder of 3-[6-(3-thienyl)benzo[b] thiophen-3-yl]pyridine hydrochloride (52 mg, 54%).
$^1$H-NMR(DMSO-$d_6$) δ: 7.69 (m, 2H), 7.87 (dd, J=1.2, 8.5Hz, 1H), 7.94 (d, J=7.9Hz, 1H), 7.98 (m, 1H), 8.02 (m, 1H), 8.16 (d, J=1.8Hz, 1H), 8.52 (s, 1H), 8.58 (m, 1H), 8.84 (d, J=5.5Hz, 1H), 9.10 (s, 1H).
IR (KBr) : 3423, 3100, 2520, 1560, 1317, 781, 688 cm$^{-1}$.

Example 32

Preparation of 3-[6-(3-furyl)benzo[b]thiophen-3-yl]pyridine hydrochloride

2M sodium carbonate aqueous solution (0.45 ml) was added to a solution of 3-(3-pyridyl)benzo[b]thiophen-6-yl-trifluoromethane sulfonate (105 mg, 0.2922 mmol) obtained in Example 13, furan-3-boronic acid (45.0 mg, 0.4022 mmol), and bistriphenylphosphine palladium (II) chloride (10.0mg, 0.01425 mmol) in THF (4 ml). The mixture was stirred for 3 hours at 80°C. The reaction mixture was cooled and diluted with ethyl acetate. Insoluble material was removed by filtration through Celite® (trademark, Wako Pure Chemical Industries, Ltd.). After removing the water layer from the filtrate, the filtrate was washed with water, then with saturated brine, and extracted with 6N hydrochloric acid. The extract was alkalinized with the addition of 50% sodium hydroxide aqueous solution, and extracted with ethyl acetate. The organic layer was washed with water, then with saturated brine, and dried with anhydrous magnesium sulfate. The solvent was evaporated under reduced pressure. The resulting residue was dissolved in diethyl ether. 1N hydrogen chloride-diethyl ether solution was added to the dissolved solution to obtain a precipitate. The precipitate was filtered to obtain a powder of 3-[6-(3-furyl)benzo[b]thiophen -3-yl]pyridine hydrochloride (72mg, 78%).
$^1$H-NMR(DMSO-$d_6$) δ: 7.10 (d, J=1.8Hz, 1H), 7.77 (dd, J=1.8, 8.6Hz, 1H), 7.79 (m, 1H), 7.93 (d, J=8.6Hz, 1H), 8.04 (dd, J=5.5, 7.9Hz, 1H), 8.17 (s, 1H), 8.32 (s, 1H), 8.40 (d, J=1.2Hz, 1H), 8.67 (d, J=7.9Hz, 1H), 8.88 (d, J=5.5Hz, 1H), 9.13 (d, J=1.8Hz, 1H). IR (KBr): 3423, 3048, 2447, 2107, 1563, 1321, 1164, 808, 784, 694, 596 cm$^{-1}$.

Example 33

Preparation of 3-[6-(2-thienyl)benzo[b]thiophen-3-yl] pyridine hydrochloride

2M sodium carbonate aqueous solution (0.45 ml) was added to a solution of 3-(3-pyridyl)benzo[b]thiophen-6-yl-trifluoromethane sulfonate (105 mg, 0.2922 mmol) obtained in Example 13, thiophene-2-boronic acid (50.0 mg, 0.3907 mmol), and bistriphenylphosphine palladium (II) chloride (10.0 mg, 0.01425 mmol) in THF (4 ml). The mixture

was stirred for 2.5 hours at 80°C. The reaction mixture was cooled and diluted with ethyl acetate. Insoluble material was removed by filtration through Celite® (trademark, Wako Pure Chemical Industries, Ltd.). After removing the water layer from the filtrate, the filtrate was washed with water, then with saturated brine, and extracted with 6N hydrochloric acid. The extract was alkalinized with the addition of 50% sodium hydroxide aqueous solution, and extracted with ethyl acetate. The organic layer was washed with water, then with saturated brine, and dried with anhydrous magnesium sulfate. The solvent was evaporated under reduced pressure. The residue obtained was dissolved in diethyl ether. 1N hydrogen chloride-diethyl ether solution was added to the dissolved solution to obtain a precipitate. The precipitate was filtered to obtain a powder of 3-[6-(2-thienyl)benzo[b]thiophen-3-yl] pyridine hydrochloride (60 mg, 62%).
$^1$H-NMR(DMSO-$d_6$) δ: 7.18 (m, 1H), 7.60 (d, J=5.5Hz, 1H), 7.64 (d, J=3.7Hz, 1H), 7.78 (d, J=8.5Hz, 1H), 7.94 (d, J=7.3Hz, 1H), 7.99 (m, 1H), 8.18 (d, J=1.8Hz, 1H), 8.47 (s, 1H), 8.59 (m, 1H), 8.86 (d, J=4.9Hz, 1H), 9.10 (s, 1H).
IR (KBr): 3435, 3080, 2604, 1557, 1316, 815, 689, 622 $cm^{-1}$.

Example 34

Preparation of 3-[6-(2-furyl)benzo[b]thiophen-3-yl]pyridine hydrochloride

2M sodium carbonate aqueous solution (0.45 ml) was added to a solution of 3-(3-pyridyl)benzo[bjthiophen-6-yl= trifluoromethane sulfonate (105 mg, 0.2922 mmol) obtained in Example 13, furan-2-boronic acid (45.0 mg, 0.4022 mmol), and bistriphenylphosphine palladium (II) chloride (10.0mg, 0.01425 mmol) in THF (4 ml). The mixture was stirred for 3 hours at 80°C. The reaction mixture was cooled and diluted with ethyl acetate. Insoluble material was removed by filtration through Celite® (trademark, Wako Pure Chemical Industries, Ltd.). After removing the water layer from the filtrate, the filtrate was washed with water, then with saturated brine, and extracted with 6N hydrochloric acid. The extract was alkalinized with the addition of 50% sodium hydroxide aqueous solution, and extracted with ethyl acetate. The organic layer was washed with water, then with saturated brine, and dried with anhydrous magnesium sulfate. The solvent was evaporated under reduced pressure. The resulting residue was dissolved in diethyl ether. 1N hydrogen chloride-diethyl ether solution was added to the solution to obtain a precipitate. The precipitate was filtered to obtain a powder of 3-[6-(2-furyl)benzo[b]thiophen-3-yl] pyridine hydrochloride (63 mg, 69%).
$^1$H-NMR(DMSO-$d_6$) δ: 6.64 (dd, J=1.8, 3.7Hz, 1H), 7.08 (d, J=3.7Hz, 1H), 7.81 (d, J=1.8Hz, 1H), 7.83 (dd, J=1.2, 8.5Hz, 1H), 7.95 (d, J=8.5Hz,1H), 7.98 (dd, J=5.5, 7.9Hz, 1H), 8.17 (s, 1H), 8.47 (s, 1H), 8.57 (d, J=7.9Hz, 1H), 8.85 (d, J=4.3Hz, 1H), 9.09 (d, J=1.8Hz, 1H).
IR (KBr): 3053, 2445, 2103, 1559, 1318, 1221, 1011, 803, 739, 691, 632$cm^{-1}$.

Example 35

Preparation of 3-(6-hexyloxybenzo[b]thiophen-3-yl)pyridine hydrochloride

A suspension of 3-(3-pyridyl)benzo[b]thiophen-6-ol (100 mg, 0.4400 mmol) obtained in Example 8, hexyl iodide (0.08 ml, 0.5432 mmol), and anhydrous potassium carbonate (80 mg, 0.5788 mmol) in DMF (5 ml) was stirred for 2 hours at 100°C, then 1 hour at 110°C. After cooling, the reaction mixture was diluted with diethyl ether. The mixture was washed with water, then with saturatedbrine, anddriedwithanhydrousmagnesiumsulfate. The solvent was evaporated under reduced pressure. The resulting residue was submitted to basic silica gel column chromatography (hexane: ethyl acetate = 9:1) to obtain 3-(6-hexyloxybenzo[b]thiophen-3-yl)pyridine (68 mg, 50%). This product was dissolved in diethyl ether. 1N hydrogen chloride-diethyl ether solutionwas added to the product solution under ice cooling to obtain a precipitate. The precipitate was filtered to obtain a powder of 3- (6-hexyloxybenzo [b] thiophen-3-yl)pyridine hydrochloride (62 mg, 41%).
$^1$H-NMR(DMSO-$d_6$) δ: 0.87 (t, J=7.0Hz, 3H), 1.31 (m, 4H), 1.41 (m, 2H), 1.74 (m, 2H), 4.05 (dd, J=6.4, 6.7Hz, 2H), 7.09 (dd, J=2.4, 9.2Hz, 1H), 7.69 (d, J=2.4Hz, 1H), 7.80 (d, J=9.2Hz, 1H), 7.99 (s, 1H), 8.03 (dd, J=5.5, 8.2Hz, 1H), 8.65 (d, J=7.9Hz, 1H), 8.86 (dd, J=1.2, 5.5Hz, 1H), 9.09 (d, J=1.8Hz, 1H).
IR (KBr): 3050, 2934, 2493, 1602, 1552, 1468, 1266, 1235, 1044, 1026, 809, 690 $cm^{-1}$.

Example 36

Preparation of 3-(6-amyloxybenzo[b]thiophen-3-yl)pyridine hydrochloride

A suspension of 3-(3-pyridyl)benzo[b]thiophen-6-ol (100 mg, 0.4400 mmol) obtained in Example 8, amyl iodide (0.07 ml, 0.5372 mmol), and anhydrous potassium carbonate (80 mg, 0.5788 mmol) in DMF (5 ml) was stirred for 4.5 hours at 110°C. After cooling, the reaction mixture was diluted with ethyl acetate. The mixture was washed with water, then with saturated brine, and dried with anhydrous magnesium sulfate. The solvent was evaporated under reduced

pressure. The resulting residue was submitted to basic silica gel column chromatography (hexane:ethyl acetate = 1: 1) to obtain 3-(6-amyloxybenzo[b]thiophen-3-yl) pyridine (116 mg, 89%). This product was dissolved in diethyl ether. 1N hydrogen chloride-diethyl ether solution was added to the solution under ice cooling to obtain a precipitate. The precipitate was filtered to obtain a powder of 3-(6-amyloxybenzo [b]thiophen-3-yl)pyridine hydrochloride (121mg, 82%).
$^1$H-NMR(DMSO-$d_6$) δ: 0.90 (t, J=7.0Hz, 3H), 1.31 (m, 4H), 1.39 (m, 4H), 1.75 (m, 2H), 4.05 (dd, J=6.4, 6.7Hz, 2H), 7.09 (dd, J=2.4, 8.9Hz, 1H), 7.69 (d, J=2.1Hz, 1H), 7.80 (d, J=9.2Hz, 1H), 7.98 (s, 1H), 8.02 (dd, J=5.5, 7.9Hz, 1H), 8.63 (d, J=7.9Hz, 1H), 8.85 (d, J=5.5Hz, 1H), 9.08 (d, J=1.5Hz, 1H).
IR (KBr): 3047, 2934, 2866, 2433, 2120, 1604, 1560, 1468, 1270, 1235, 1020, 828, 799, 691 $cm^{-1}$.

Example 37

Preparation of 3-(6-butyloxybenzo[b]thiophen-3-yl)pyridine hydrochloride

A suspension of 3-(3-pyridyl)benzo[b]thiophen-6-ol (100 mg, 0.4400 mmol) obtained in Example 8, butyl iodide (0.06 ml, 0.5217 mmol), and anhydrous potassium carbonate (80 mg, 0.5788 mmol) in DMF (5 ml) was stirred for 22.5 hours at 110°C. The reaction mixture was cooled. After the addition of butyl iodide (0.06 ml, 0.5217 mmol), the mixture was stirred for 5 hours at 110°C. After cooling, the reaction mixture was diluted with ethyl acetate. The mixture was washed with water, then with saturated brine, and dried with anhydrous magnesium sulfate. The solvent was evaporated under reduced pressure. The resulting residue was submitted to basic silica gel column chromatography (hexane:ethyl acetate = 1:1) to obtain 3-(6-butyloxybenzo[b]thiophen-3-yl)pyridine (115 mg, 92%). This product was dissolved in diethyl ether. 1N hydrogen chloride-diethyl ether solution was added to the product solution under ice cooling to obtain a precipitate. The precipitate was filtered to obtain a powder of 3-(6-butyloxybenzo[b]thiophen -3-yl)pyridine hydrochloride (106mg, 75%).
$^1$H-NMR(DMSO-$d_6$) δ: 0.94 (t, J=7.3Hz, 3H), 1.45 (m, 2H), 1.73 (m, 2H), 4.06 (dd, J=6.4, 6.7Hz, 2H), 7.09 (dd, J=2.4, 8.9Hz, 1H), 7.70 (d, J=2.4Hz, 1H), 7.79 (d, J=8.9Hz, 1H), 7.96 (s, 1H), 7.98 (dd, J=5.5, 7.9Hz, 1H), 8.59 (d, J=8.2Hz, 1H), 8.84 (dd, J=1.2, 5.5Hz, 1H), 9.07 (d, J=1.8Hz, 1H).
IR (KBr) : 3052, 2957, 2932, 2870, 2552, 2116, 1604,1550, 1466, 1272, 1235, 1046, 1010, 827, 805, 688, 650 $cm^{-1}$.

Example 38

Preparation of 1-{2-[3-(3-pyridyl)benzo[b]thiophen-6-yl] phenyl}-1-ethanone hydrochloride

2M sodium carbonate aqueous solution (1.20 ml) was added to a solution of 3-(3-pyridyl)benzo[b]thiophen-6-yl-trifluoromethane sulfonate (200 mg, 0.5566 mmol) obtained in Example 13, 2-acetylphenyl boronic acid (100 mg, 0.6099 mmol), and bistriphenylphosphine palladium (II) chloride (20.0 mg, 0.02849 mmol) in THF (10 ml). The mixture was stirred for 1.5 hours at 80°C. The reaction mixture was cooled and diluted with diethyl ether. Insoluble material was removed by filtration through Celite® (trademark, Wako Pure Chemical Industries, Ltd.). After removing the water layer from the filtrate, the filtrate was washed with water, then with saturated brine, and extracted with 2N hydrochloric acid. The extract was alkalinized with the addition of diluted sodium hydroxide aqueous solution, and extracted with diethyl ether. The organic layer was washed with water, then with saturated brine, and dried with anhydrous magnesium sulfate. The solvent was evaporated under reduced pressure. The residue obtained was dissolved in diethyl ether. 1N hydrogen chloride-diethyl ether solution was added to the dissolved solution to obtain a precipitate. The precipitate was filtered to obtain a powder of 1-{2-[3-(3-pyridyl)benzo [b] thiophen-6-yl]phenyl}-1-ethanone hydrochloride (144 mg, 71%).
$^1$H-NMR(DMSO-$d_6$) δ: 2.22 (s, 3H), 7.39 (dd, J=1.8, 8.5Hz, 1H), 7.49 (d, J=7.6Hz, 1H), 7.53 (dd, J=1.2, 7.6Hz, 1H), 7.62 (dt, J=1.2, 7.6Hz, 1H), 7.69 (d, J=7.6Hz, 1H), 7.97 (d, J=8.2Hz, 1H), 8.03, (dd, J=5.5, 7.9Hz, 1H), 8.11 (d, J=1.2Hz, 1H), 8.25 (s, 1H), 8.67 (d, J=8.2Hz, 1H), 8.88 (d, J=4.28Hz, 1H), 9.14 (s, 1H).
IR (KBr): 3046, 3262, 2100, 1559, 1357, 1247, 825, 775, 692, 622 $cm^{-1}$.

Example 39

Preparation of 1-{3-[3-(3-pyridyl)benzo[b]thiophen-6-yl] phenyl}-1-ethanone

2M sodium carbonate aqueous solution (1.10 ml) was added to a solution of 3-(3-pyridyl)benzo[b]thiophen-6-yl-trifluoromethane sulfonate (177 mg, 0.4926 mmol) obtained in Example 13, 3-acetylphenyl boronic acid (89 mg, 0.5428 mmol), and bistriphenylphosphine palladium (II) chloride (17.0 mg, 0.02422 mmol) in THF (9 ml). The mixture was stirred for 3 hours at 80°C. The reaction mixture was cooled and, after the addition of 3-acetylphenyl boronic acid (16 mg, 0.09757 mmol), the mixture was stirred for 1 hour at 80°C. The reaction mixture was cooled and diluted with

diethyl ether. Insoluble material was removed by filtration through Celite® (trademark, Wako Pure Chemical Industries, Ltd.). After removing the water layer from the filtrate, the filtrate was washed with water, then with saturated brine, and extracted with 2N hydrochloric acid. The extract was alkalinized with the addition of diluted sodium hydroxide aqueous solution, and extractedwith diethyl ether. The organic layer was washed with water, then with saturated brine, and dried with anhydrous magnesium sulfate. The solvent was evaporated under reduced pressure. The resulting residue was purified by crystallizing in ethyl acetate-hexane to obtain a powder of 1-{3-[3-(3-pyridyl)benzo[b]thiophen-6-yl]phenyl}-1-ethanone (90 mg, 74%).
$^1$H-NMR($CDCl_3$) δ: 2.67 (s, 3H), 7.44 (dd, J=5.2, 7.9Hz, 1H), 7.51 (s, 1H), 7.56 (t, J=7.6Hz, 1H), 7.67 (dd, J=1.5, 8.2Hz, 1H), 7.86 (d, J=8.24Hz, 1H), 7.91-7.96 (m, 3H), 8.16 (d, J=1.52Hz, 1H), 8.25 (s, 1H), 8.66 (d, J=3.1Hz, 1H), 8.87 (s, 1H).
IR (KBr): 3076, 1673, 1406, 1354, 1267, 1235, 1026, 830, 800, 710, 693, 585 $cm^{-1}$.

Example 40

Preparation of 3-(6-isobutyloxybenzo[b]thiophen-3-yl) pyridine hydrochloride

A suspension of 3-(3-pyridyl)benzo[b]thiophen-6-ol (100 mg, 0.4400 mmol) obtained in Example 8, isobutyl iodide (0.10 ml, 0.8695 mmol), and anhydrous potassium carbonate (120 mg, 0.9360 mmol) in DMF (5 ml) was stirred for 5.5 hours at 110°C. The reaction mixture was cooled, after the addition of isobutyl iodide (0.10 ml, 0.8695 mmol), and was stirred for 15.5 hours at 110°C. After cooling, the reaction mixture was diluted with ethyl acetate. The mixture was washed with water, then with saturated brine, and dried with anhydrous magnesium sulfate. The solvent was evaporated under reduced pressure. The resulting residue was submitted to silica gel column chromatography (hexane: ethyl acetate = 4:1) to obtain 3- (6-isobutyloxybenzo[b]thiophen-3-yl)pyridine (62 mg, 50%). This product was dissolved in diethyl ether. 1N hydrogen chloride-diethyl ether solution was added to the solution under ice cooling to obtain a precipitate. The precipitate was filtered to obtain a powder of 3- (6-isobutyloxybenzo [b] thiophen -3-yl)pyridine hydrochloride (51 mg, 36%).
$^1$H-NMR(DMSO-$d_6$) δ: 0.99 (d, J=6.7Hz, 6H) , 2.04 (m, 1H), 3.84 (d, J=6.7Hz, 2H), 7.10 (dd, J=2.4, 8.9Hz, 1H), 7.70 (d, J=2.4Hz, 1H), 7.80 (d, J=8.9Hz, 1H), 7.98 (s, 1H), 8.02 (dd, J=5.5, 7.9Hz, 1H), 8.63 (dt, J=1.8, 8.2Hz, 1H), 8.86 (dd, J=1.2, 5.5Hz, 1H), 9.09 (d, J=1.8Hz, 1H).
IR (KBr) : 3012, 2917, 2871, 1602, 1548, 1455, 1277, 1256, 1225, 1050, 1028, 806, 687, 617 $cm^{-1}$.

Example 41

Preparation of 3- (6-propyloxybenzo [b] thiophen-3-yl)pyridine hydrochloride

A suspension of 3-(3-pyridyl)benzo[b]thiophen-6-ol (100 mg, 0.4400 mmol) obtained in Example 8, propyl bromide (0.10 ml, 1.101 mmol), and anhydrous potassium carbonate (160 mg, 1.158 mmol) in DMF (5 ml) was stirred for 3 hours at 110°C. After cooling, the reaction mixture was diluted with ethyl acetate. The mixture was washed with water, then with saturated brine, and dried with anhydrous magnesium sulfate. The solvent was evaporated under reduced pressure. The resulting residue was submitted to silica gel column chromatography (hexane:ethyl acetate = 4:1) to obtain 3-(6-propyloxybenzo[b]thiophen-3-yl)pyridine (76mg, 64%). This product was dissolved in ethanol. 1N hydrogen chloride-diethyl ether solution was added to the product solution under ice cooling to obtain a precipitate. The precipitate was filtered to obtain a powder of
3-(6-propyloxybenzo[b]thiophen-3-yl)pyridine hydrochloride (50 mg, 42%).
$^1$H-NMR(DMSO-$d_6$) δ: 1.00 (t, J=7.3Hz, 3H) 1.76 (m, 2H), 4.02(t, J=6.7Hz, 2H), 7.10 (dd, J=2.4, 8.9Hz, 1H), 7.70 (d, J=2.4Hz, 1H), 7.80 (d, J=8.9Hz, 1H), 7.99 (s, 1H), 8.04 (dd, J=5.5, 7.9Hz, 1H), 8.66 (dt, J=1.8, 8.4Hz, 1H), 8.87 (dd, J=1.2, 5.5Hz, 1H), 9.10(d, J=1.8Hz, 1H).
IR (KBr) : 3025, 2935, 2481, 1607, 1552, 1509, 1467, 1448, 1414, 1388, 1363, 1329, 1310, 1270, 1235, 892, 830, 804, 690, 649, 622 $cm^{-1}$.

Example 42

Preparation of 3-[6-(1,3-benzodioxol-5-yl)benzo[b]thiophen -3-yl]pyridine hydrochloride

2M sodium carbonate aqueous solution (1.35 ml) was added to a solution of 3-(3-pyridyl)benzo[b]thiophen-6-yl= trifluoromethane sulfonate (225 mg, 0.6261 mmol) obtained in Example 13, 3,4-methylenedioxyphenyl boronic acid (125 mg, 0.7533mmol), and bistriphenylphosphine palladium (II) chloride (22.0 mg, 0.03134 mmol) in THF (5 ml). The mixture was stirred for 4 hours at 80°C. After cooling, 3,4-methylenedioxyphenyl boronic acid (52.0 mg, 0.3134 mmol) and bistriphenylphosphine palladium (II) chloride (10.0 mg, 0.01425 mmol) were added. The mixture was stirred

for 1.5 hours at 80°C. The reaction mixture was cooled and diluted with diethyl ether. Insoluble material was removed by filtration through Celite® (trademark, Wako Pure Chemical Industries, Ltd.). After removing the water layer from the filtrate, the filtrate was extracted with ethyl acetate, and washed with water, and then with saturated brine. The organic layer was washed with water, then with saturated brine, and dried with anhydrous magnesium sulfate. The solvent was evaporated under reduced pressure. The residue obtained was dissolved in diethyl ether-ethyl acetate. 6N hydrochloric acid aqueous solution was added to the dissolved solution to obtain a precipitate. The precipitate was filtered to obtain a powder of 3-[6-(1,3-benzodioxole-5-yl)benzo[b]thiophen-3-yl] pyridine hydrochloride (151 mg, 65%).
$^1$H-NMR(DMSO-$d_6$) δ: 6.08 (s, 2H), 7.03 (d, J=7.9Hz, 1H), 7.27 (dd, J=1.8, 8.2Hz, 1H), 7.37 (d, J=1.8Hz, 1H), 7.75 (dd, J=1.8, 8.6Hz, 1H), 7.94 (d, J=8.6Hz, 1H), 7.97 (dd, J=5.5, 7.9Hz, 1H), 8.16 (s, 1H), 8.39(d, J=1.8Hz, 1H), 8.58 (d, J=8.2Hz, 1H), 8.84 (d, J=5.5Hz, 1H), 9.09 (d, J=2.1Hz. 1H).
IR (KBr) : 3477, 3055, 2619, 1559, 1502, 1469, 1256, 1227, 1042, 934, 801, 685 $cm^{-1}$.

Example 43

Preparation of 3-(6-ethyloxybenzo[b]thiophen-3-yl)pyridine hydrochloride

A suspension of 3-(3-pyridyl)benzo[b]thiophen-6-ol (250 mg, 1.100 mmol) obtained in Example 8, ethyl bromide (0.10 ml, 1.340 mmol), and anhydrous potassium carbonate (200 mg, 1.447 mmol) in DMF (10 ml) was stirred for 3 hours at 110°C. After cooling, the reaction mixture was diluted with ethyl acetate. The mixture was washed with water, then with saturated brine, and dried with anhydrous magnesium sulfate. The solvent was evaporated under reduced pressure. The resulting residue was submitted to silica gel column chromatography (hexane:ethyl acetate = 4:1) to obtain 3-(6-ethyloxybenzo[b]thiophen-3-yl) pyridine (240 mg, 85%). This product was dissolved in ethanol. 1N hydrogen chloride-diethyl ether solution was added to the product solution under ice cooling to obtain a precipitate. The precipitate was filtered to obtain a powder of 3- (6-ethyloxybenzo [b]thiophen-3-yl)pyridine hydrochloride (218 mg, 68%).
$^1$H-NMR(DMSO-$d_6$) δ: 1.36 (t, J=7.0Hz, 3H), 4.12 (q, J=7.0Hz, 2H), 7.09(dd, J=2.4, 8.8Hz, 1H), 7.69 (d, J=2.4Hz, 1H), 7.81 (d, J=8.8Hz, 1H), 8.00 (s, 1H), 8.06 (dd, J=5.5, 7.9Hz, 1H), 8.68 (d, J=7.9Hz, 1H), 8.88(d, J=5.5Hz, 1H), 9.11 (s, 1H).
IR (KBr) : 3044, 2984, 2919, 2874, 2362, 2109, 1982, 1598, 1555, 1508, 1468, 1455, 1280, 1231, 1056, 941, 802, 683 $cm^{-1}$.

Example 44

Preparation of 1-{4-[3-(3-pyridyl)benzo[b]thiophen-6-yl] phenyl}-1-ethanone

2M sodium carbonate aqueous solution (1.20 ml) was added to a solution of 3-(3-pyridyl)benzo[b]thiophen-6-yl= trifluoromethane sulfonate (200 mg, 0.5566 mmol) obtained in Example 13, 4-acetylphenyl boronic acid (100 mg, 0.6099 mmol), and bistriphenylphosphine palladium (II) chloride (20.0 mg, 0.02849 mmol) in THF (10 ml). The mixture was stirred for 3 hours at 80°C. The reaction mixture was cooled and diluted with diethyl ether. Insoluble material was removed by filtration through Celite® (trademark, Wako Pure Chemical Industries, Ltd.). After removing the water layer from the filtrate, the filtrate was washed with water, then with saturated brine. 6N hydrochloric acid was added and the precipitate formed was collected by filtration. The precipitate was dissolved in 5N sodium hydroxide, extracted with diethyl ether, and washed with water and then with saturated brine. The organic layer was dried with anhydrous magnesium sulfate. The solvent was evaporated to obtain a powder of 1-{4-[3-(3-pyridyl)benzo[b]thiophen-6-yl]phenyl}-1-ethanone (69 mg, 38%).
$^1$H-NMR($CDCl_3$) δ:2.64 (s, 3H), 7.43 (dd, J=4.6, 7.6Hz, 1H), 7.52 (s, 1H), 7.67 (dd, J=1.8, 8.6, 1H), 7.75 (d, J=8.2, 2H), 7.89-7.92 (m, 2H), 8.04 (d, J=8.6Hz, 2H), 8.16 (d, J=1.2Hz, 1H), 8.66 (s, 1H), 8.86 (s, 1H).
IR (KBr): 3060, 3027, 1678, 1604, 1358, 1268, 819, 715 $cm^{-1}$.

Example 45

Preparation of 3-[6-(N,N-dimethyl-2-aminoethyloxy)benzo[b] thiophen-3-yl]pyridine dimaleate

A suspension of 3-(3-pyridyl)benzo[b]thiophen-6-ol (200 mg, 0.8800 mmol) obtained in Example 8, 2-dimethylaminoethyl chloride hydrochloride (150 mg, 1.041 mmol), and anhydrous potassium carbonate (280mg, 2.026mmol) inDMF (10ml) was stirred for 24 hours at 80°C. After cooling, the reaction mixture was diluted with ethyl acetate. The mixture was washed with water, then with saturated brine, and dried with anhydrous magnesium sulfate. The solvent was evaporated under reduced pressure. The resulting residue was submitted to basic silica gel column chromatography (hexane:ethylacetate=4:1) to obtain 3-[6-(N,N-dimethyl-2-aminoethyloxy)benzo[b]thiophen-3-yl]pyridine (110 mg, 42%) . This product was dissolved in ethanol. 1Mmaleic acid solution in ethanol was added to the product solution under ice cooling to obtain a precipitate. The precipitate was filtered to obtain a powder of 3-[6-(N,N-dimethyl-2-aminoethyloxy)benzo[b]thiophen-3-yl]pyridine dimaleate (92 mg, 20%).
$^1$H-NMR(DMSO-$d_6$) δ: 2.88(s, 6H), 3.57(dd, J=4.9, 5.2Hz, 2H), 4.41(dd, J=4.9, 5.2Hz, 2H), 6.13(s, 4H), 7.14(dd, J=2.4, 8,8Hz, 1H), 7.55(dd, J=4.9, 7.9Hz, 1H), 7.76(d, J=2.4Hz, 1H), 7.79(d, J=8.8Hz, 1H), 7.82(s, 1H), 8.02(ddd, J=1.8, 2.1, 7.9Hz, 1H), 8.63(dd, J=1.5, 4.9Hz, 1H), 8.81(d, J=1.8Hz, 1H).

Example 46

Preparation of 3-(6-isobutylbenzo[b]thiophen-3-yl)pyridine hydrochloride

0.5 M isobutyl zinc bromide solution in THF (1.2 ml) was added to a solution of 3-(3-pyridyl)benzo[b]thiophen-6-yl= trifluoromethane sulfonate (200 mg, 0.5566 mmol) obtained in Example 13 in THF (5 ml) in argon atmosphere. After the addition of tetrakistriphenylphosphine palladium (0) (32 mg, 0.02769 mmol), the mixture was stirred for 1.5 hours at 80°C. The reaction solution was cooled, saturated ammonium chloride aqueous solution was added and the reaction solution was stirred for appropriate time, and extracted with ethyl acetate. The organic layer was washed with water, then with saturated brine, and dried with anhydrous magnesium sulfate. The solvent was evaporated under reduced pressure. The resulting residue was submitted to silica gel column chromatography (hexane:ethyl acetate = 3:2) to obtain 3-(6-isobutylbenzo[b]thiophen-3-yl)pyridine (48 mg, 32%). This product was dissolved in ethanol. 1N hydrogen chloride-diethyl ether solution was added to the product solution under ice cooling to obtain a precipitate. The precipitate was filtered to obtain a powder of 3-(6-isobutylbenzo[b]thiophen -3-yl)pyridine hydrochloride (48 mg, 28%).
$^1$H-NMR (DMSO-$d_6$) δ: 0.88 (d, J=6.7Hz, 6H), 1.92 (m, J=6.7Hz, 1H), 2.61(d, J=6.7Hz, 2H), 7.31 (dd, J=1.2, 8.5Hz, 1H), 7.84 (d, J=8.5Hz, 1H), 7.90 (s, 1H), 8.03 (dd, J=5.5, 7.9Hz, 1H), 8.11 (s, 1H), 8.65 (d, J=7.9Hz, 1H), 8.86 (d, J=5.5Hz, 1H), 9.11 (br s, 1H).
IR (KBr): 3437, 3081, 2922, 1617, 1563, 1500, 1459, 803 $cm^{-1}$.

Example 47

Preparation of 3-[6-(4-methylthiophenyl)benzo[b]thiophen-3-yl]pyridine hydrochloride

2M sodium carbonate aqueous solution (0.450 ml) was added to a solution of 3-(3-pyridyl)benzo[b]thiophen-6-yl-trifluoromethane sulfonate (105 mg, 0.2922 mmol) obtained in Example 13, 4-methylthiophenyl boronicacid (67mg, 0.3988mmol), and bistriphenylphosphine palladium (II) chloride (10.0 mg, 0.01425 mmol) in THF (4 ml). The mixture was stirred for 3 hours at 80°C. The reaction mixture was cooled and diluted with ethyl acetate. Insoluble material was removed by filtration through Celite® (trademark, Wako Pure Chemical Industries, Ltd.). After removing the water layer from the filtrate, the filtrate was washed with water, then with saturated brine, and extracted with 6N hydrochloric acid. The extract was alkalinized with the addition of 50% sodium hydroxide aqueous solution, and extracted with ethyl acetate. The organic layer was washed with water, then with saturated brine, and dried with anhydrous magnesium sulfate. The solvent was evaporated under reduced pressure. The residue obtained was dissolved in diethyl ether. 1N hydrogen chloride-diethyl ether solution was added to the dissolved solution to obtain a precipitate. The precipitate was filtered to obtain a powder of 3-[6-(4-methylthiophenyl)benzo[b]thiophen-3-yl]pyridine hydrochloride (89 mg, 82%).
$^1$H-NMR (DMSO-$d_6$) δ: 2.52 (s, 3H), 7.38 (d, J=8.6Hz, 2H), 7.75 (d, J=7.9Hz, 2H), 7.79 (dd, J=1.8, 8.6Hz, 1H), 7.98 (d, J=7.9Hz, 1H), 8.03(dd, J=6.1, 7.3Hz, 1H), 8.21 (s, 1H), 8.45 (d, J=1.2Hz, 1H), 8.65 (br d, J=7.9Hz, 1H), 8.88 (d, J=5.5Hz, 1H), 9.13 (br s, 1H).
IR (KBr): 3050, 2367, 2107, 1561, 1320, 807 $cm^{-1}$.

Example 48

Preparation of 3-{[6-(2-trifluoromethyl)phenyl]benzo[b] thiophen-3-yl}pyridine hydrochloride

2M sodium carbonate aqueous solution (1.20 ml) was added to a solution of 3-(3-pyridyl)benzo[b]thiophen-6-yl-trifluoromethane sulfonate (190 mg, 0.5287 mmol) obtained in Example 13, 2-trifluoromethylphenylboronicacid (110 mg, 0.5792 mmol), and bistriphenylphosphine palladium (II) chloride (20.0 mg, 0.02849 mmol) in THF (10 ml). The mixture was stirred for 3.5 hours at 80°C. After cooling, 2-trifluoromethylphenyl boronic acid (40 mg, 0.2106 mmol) and bistriphenylphosphine palladium (II) chloride (10.0 mg, 0.01425 mmol) were added. The mixture was stirred for 20.5 hours at 80°C. The reaction mixture was cooled and diluted with diethyl ether. Insoluble material was removed

by filtration through Celite® (trademark, Wako Pure Chemical Industries, Ltd.). After removing the water layer from the filtrate, the filtrate was washed with water, then with saturated brine, and extracted with 6N hydrochloric acid. The extract was alkalinized with the addition of 50% sodium hydroxide aqueous solution, and extracted with diethyl ether. The organic layer was washed with water, then with saturated brine, and dried with anhydrous magnesium sulfate. The solvent was evaporated under reduced pressure. The resulting residue was dissolved in diethyl ether. 1N hydrogen chloride-diethyl ether solution was added to the solution to obtain a precipitate. The precipitate was filtered to obtain a powder of 3-{[6-(2-trifluoromethyl) phenyl]benzo[b]thiophen-3-yl}pyridine hydrochloride (112 mg, 54%).
$^1$H-NMR (DMSO-$d_6$) δ: 7.43 (d, J=10Hz, 1H), 7.48 (d, J=10Hz, 1H), 7.66 (t, J=10Hz, 1H), 7.76 (t, J=10Hz, 1H), 7.87 (d, J=5Hz, 1H), 7.79-8.01 (m, 2H), 8.11 (s, 1H), 8.25 (s, 1H), 8.62 (d, J=5Hz, 1H), 8.86 (d, J=5Hz, 1H), 9.12 (s, 1H).
IR (KBr): 3067, 2345, 2072, 1561, 1313, 1179, 1126, 1109, 1036, 802, 767, 688, 624 $cm^{-1}$.

Example 49

Preparation of isopropyl{3-[3-(3-pyridyl)benzo[b]thiophen-6-yl]phenyl}amine

Cyano sodium borohydride (30 mg, 0.4774 mmol) and acetic acid (50 mg, 0.8326 mmol) were added to a suspension of 3-[3-(3-pyridyl)benzo[b]thiophen-6-yl]phenylamine (100 mg, 0.3307 mmol) obtained in Example 23 and acetone (50 mg, 0.8609 mmol) in methanol (2 ml). The mixture was stirred for 23 hours at room temperature. After the reaction mixture was added with dilute hydrochloric acid up to pH 2, and further stirred for 7 hours at room temperature. The solvent was removed by vacuum evaporation. The water layer was washed with chloroform, alkalinized with the addition of dilute sodium hydroxide solution, and extracted with chloroform. The organic layer was washed with saturated brine and dried with anhydrous sodium sulfate. The solvent was evaporated under reduced pressure. The resulting residue was submitted to basic silica gel column chromatography (hexane:ethyl acetate = 1:1). The eluate was neutralized with dilute hydrochloric acid and saturated sodium bicarbonate solution to form a precipitate. The precipitate was filtered to obtain isopropyl{3-[3-(3-pyridyl)benzo[b] thiophen-6-yl]phenyl}amine (30 mg, 26%).
$^1$H-NMR ($CDCl_3$) δ: 1.24 (d, J=6.1Hz, 6H), 3.59 (br s, 1H), 3.71 (m, 1H), 6.59 (dd, J=1.5, 7.4Hz, 1H), 6.84 (dd, J=1.8, 2.1Hz, 1H), 6.96 (d, J=7.6Hz, 1H), 7.23-7.24 (m, 2H), 7.42 (m, 1H), 7.46 (s, 1H), 7.62 (dd, J=1.8, 8.5Hz, 1H), 7.86 (d, J=8.6Hz, 1H), 7.91 (d, J=7.9Hz, 1H), 8.10 (d, J=1.5Hz, 1H), 8.65 (br s, 1H), 8.87 (br s, 1H).
IR (KBr) : 3304, 3030, 2963, 1601, 1510, 1327, 1226, 1173, 823, 755, 713 $cm^{-1}$.

Example 50

Preparation of 3-(6-cyclohexylbenzo[b]thiophen-3-yl)pyridine hydrochloride

Under argon atmosphere 0.5 M cyclohexyl zinc bromide solution in THF (1.2 ml) was added to a solution of 3-(3-pyridyl)benzo[b]thiophen-6-yl= trifluoromethane sulfonate (100 mg, 0.2783 mmol) obtained in Example 13 in THF (2 ml). After further addition of tetrakistriphenylphosphine palladium (0) (16 mg, 0.01385 mmol), the mixture was stirred for 2 hours at 80°C. The reaction mixture was cooled, saturated ammonium chloride aqueous solution was added and the mixture was stirred for appropriate time, and then extracted with ethyl acetate. The organic layer was washed with water, then with saturated brine, and dried with anhydrous magnesium sulfate. The solvent was evaporated under reduced pressure. The resulting residue was submitted to silica gel column chromatography (hexane:ethyl acetate = 2:1) to obtain 3-(6-cyclohexylbenzo[b]thiophen-3-yl)pyridine (45 mg, 55%). This product was dissolved in ethanol. 1N hydrogen chloride-diethyl ether solution was added to the product solution under ice cooling to obtain a precipitate. The precipitate was filtered to obtain a powder of 3- (6-cyclohexylbenzo[b]thiophen -3-yl)pyridine hydrochloride (40 mg, 44%).
$^1$H-NMR (DMSO-$d_6$) δ: 1.27 (m, 1H), 1.38-1.50 (m, 4H), 1.71-1.85 (m, 5H), 2.66 (m, 1H), 7.38 (d, J=8.6Hz, 1H), 7.82 (d, J=8.6Hz, 1H), 7.96 (s, 1H), 8.01 (dd, J=5.5, 7.9Hz, 1H), 8.10 (s, 1H), 8,62 (d, J=7.9Hz, 1H), 8.85(d, J=5.5Hz, 1H), 9.09 (s, 1H).
IR (KBr): 3029, 2922, 2372, 2109, 1556, 1449, 1312, 813, 685, 621 $cm^{-1}$.

Example 51

Preparation of 3-[6-(3-isopropyloxyphenyl)benzo[b]thiophen -3-yl)pyridine hydrochloride

A suspension of 3-[3-(3-pyridyl)benzo[b]thiophen-6-yl] phenol (300 mg, 0.9889 mol) obtained in Example 17, isopropyl bromide (0.10ml, 1.0692mmol), and anhydrous potassium carbonate (160 mg, 1.1577 mmol) in DMF (15 ml) was stirred for 22 hours at 100°C. The reaction mixture was cooled, and after addition of isopropyl bromide (0.10 ml, 1.0692 mmol) and anhydrous potassium carbonate (160 mg, 1.1577 mmol), the mixture was stirred for 21 hours at

100°C. After cooling, the reaction mixture was diluted with ethyl acetate. The mixture was washed with water, then with saturated brine, and dried with anhydrous magnesium sulfate. The solvent was evaporated under reduced pressure. The resulting residue was submitted to basic silica gel column chromatography (hexane : ethyl acetate=19:1) to obtain 3-[6-(3-isopropyloxyphenyl)benzo[b]thiophen-3-yl]pyridine (280 mg, 82%). This product was dissolved in ethanol. 1N hydrogen chloride-diethyl ether solution was added to the product solution under ice cooling to obtain a precipitate. The precipitate was filtered to obtain a powder of 3-[6-(3-isopropyloxyphenyl)benzo[b]thiophen-3-yl]pyridine hydrochloride (180 mg, 48%).
$^1$H-NMR (DMSO-$d_6$) δ: 1.29 (d, J=5.8Hz, 6H), 4.75 (sept, J=5.8Hz, 1H), 6.95 (dd, J=2.1, 7.9Hz, 1H), 7.28 (m, 1H), 7.31 (d, J=7.6Hz, 1H), 7.38 (t, J=7.9Hz, 1H), 7.80 (dd, J=1.5, 8.6Hz, 1H), 7.98 (d, J=8.6Hz, 1H), 8.06 (dd, J=5.5, 7.9Hz, 1H), 8.24 (s, 1H), 8.47 (d, J=1.8Hz/ 1H), 8.70 (d, J=7.9Hz, 1H), 8.90 (d, J=4.6Hz, 1H), 9.15 (d, J=1.8Hz, 1H).
IR (KBr): 3421, 2975, 1577, 1543, 1458, 1204, 1116, 778, 683 $cm^{-1}$.

Example 52

Preparation of 3-(6-propylbenzo[b]thiophen-3-yl)pyridine hydrochloride

Under argon atmosphere 0.5Mn-propylzinc bromide solution in THF (1.8 ml) was added to a solution of 3-(3-pyridyl)benzo[b]thiophen-6-yl= trifluoromethane sulfonate (150 mg, 0.4174 mmol) obtained in Example 13 in THF (3 ml). After the addition of tetrakistriphenylphosphine palladium (0) (24 mg, 0.02077 mmol), the mixture was stirred for 1 hour at 80°C. After cooling, the reaction mixture was added saturated ammonium chloride aqueous solution, stirred for appropriate time, and then extracted with ethyl acetate. The organic layer was washed with water, then with saturated brine, and dried with anhydrous magnesium sulfate. The solvent was evaporated under reduced pressure. The resulting residue was submitted to silica gel column chromatography (hexane:ethyl acetate = 2:1) to obtain a colorless oily product of 3-(6-propylbenzo[b]thiophen-3-yl) pyridine (54mg, 51%). This product was dissolved in ethanol. 1N hydrogen chloride-diethyl ether solution was added to the product solution under ice cooling to obtain a precipitate. The precipitate was filtered to obtain a powder of 3-(6-propylbenzo[b]thiophen-3-yl)pyridine hydrochloride (49 mg, 41%).
$^1$H-NMR (DMSO-$d_6$) δ: 0.91 (t, J=7.3Hz, 3H), 1.66 (sext, J=7.3Hz, 2H), 2.71 (t, J=7.3Hz, 2H), 7.34 (d, J=8.6Hz, 1H), 7.83 (d, J=8.6Hz, 1H), 7.93 (s, 1H), 8.00 (m, 1H), 8.09 (d, J=2.4Hz, 1H), 8.61 (m, 1H), 8.85(br d, J=4.9Hz, 1H), 9.09 (s, 1H).
IR (KBr) : 3421, 3045, 2925, 2471, 2113, 1559, 1315, 1261, 1223, 808, 691$cm^{-1}$.

Example 53

Preparation of 3-[6-(3-nitrophenyl)benzo[b]thiophen-3-yl] pyridine

2M sodium carbonate aqueous solution (1.00 ml) was added to a solution of 3-(3-pyridyl)benzo[b]thiophen-6-yl= trifluoromethane sulfonate (206 mg, 0.5733 mmol) obtained in Example 13, 3-nitrophenyl boronic acid (105 mg, 0.6290 mmol), and bistriphenylphosphine palladium (II) chloride (20.0 mg, 0.02849 mmol) in THF (10 ml). The mixture was stirred for 5 hours at 80°C. The reaction mixture was cooled and diluted with diethyl ether. Insoluble material was removed by filtration through Celite® (trademark, Wako Pure Chemical Industries, Ltd.). After removing the water layer from the filtrate, the filtrate was washed with water, then with saturated brine, and dried with anhydrous magnesium sulfate. The solvent was evaporated under reduced pressure. The resulting residue was crystallized in ethyl acetate-hexane to obtain a powder of 3-[6- (3-nitrophenyl) benzo[b]thiophen-3-yl]pyridine (72 mg, 38%).
$^1$H-NMR ($CDCl_3$) δ: 7.45 (dd, J=4.9, 7.6Hz, 1H), 7.55 (s, 1H), 7.63 (t, J=7.9Hz, 1H), 7.67 (dd, J=1.8, 8.5Hz, 1H) , 7.91 (ddd, J=1.5, 1.8, 7.9Hz, 1H), 7.94 (d, J=8.5Hz, 1H), 7.98 (m, 1H), 8.17 (d, J=1.8Hz, 1H), 8.21 (m, 1H), 8.52 (t, J=1.8Hz, 1H), 8.66 (dd, J=1.5, 4.9Hz, 1H), 8.86 (d, J=1.8Hz, 1H).
IR (KBr): 3096, 1529, 1345, 813, 777, 734, 712 $cm^{-1}$.

Example 54

Preparation of isopropyl[3-(3-pyridyl)benzo[b]thiophen-6-yl] amine

Acetic acid palladium (II) (33 mg, 0.1470 mmol) was added to a suspension of 3-(3-pyridyl)benzo[b]thiophen-6-yl= trifluoromethane sulfonate (2.63 g, 7.319 mmol) obtained in Example 13, benzophenone imine (1.47 ml, 8.760 mmol), caesium carbonate (3.34 g, 10.25 mmol), and (R)-(+)-2,2'-bis (diphenylphosphino)-1,1'-binaphthyl (BINAP) (137 mg, 0.2200 mmol) in THF (25 ml). The mixture was stirred for 15.5 hours at 65°C. The mixture was cooled and, after the addition of 2N hydrochloric acid, further stirred for 3 hours at room temperature. The reaction mixture was washed with ethyl acetate. The water layer was made alkaline with dilute sodium hydroxide aqueous solution and

extracted with ethyl acetate. The organic layer was washed with water, then with saturated brine, and dried with anhydrous sodium sulfate. The solvent was evaporated under reduced pressure. The residue obtained was submitted to silica gel column chromatography (hexane:ethyl acetate = 3:1-3:2). The resulting pale yellow oily substance was allowed to stand overnight in a refrigerator to obtain a pale yellow solid of 3-(3-pyridyl)benzo[b]thiophen-6-yl amine (1.38 g, 83%).
$^1$H-NMR (DMSO-$d_6$) δ: 5.33 (br s, 2H), 6.75 (dd, J=1.8, 8.6Hz, 1H), 7.08(d, J=1.8Hz, 1H), 7.42 (s, 1H), 7.49-7.52 (m, 2H), 7.96 (dt, J=2.1, 7.9Hz, 1H), 8.58 (dd, J=1.5, 4.9Hz, 1H), 8.76 (dd, J=0.9, 2.4Hz, 1H).
IR (KBr) : 3197, 1604, 1524, 1465, 1426, 1349, 1320, 1297, 1243, 1188, 1038, 812, 766, 714 $cm^{-1}$.

Acetic acid (3 droplets) and cyano sodium borohydride (16 mg, 0.2548 mmol) were added to a solution of the 3- (3-pyridyl)benzo[b]thiophen-6-yl amine (116mg, 0.5126 mmol) obtained above in methanol (2 ml). After acetone (0.045 ml, 0.6129 mmol) was further added, the mixture was reacted for 19 hours at room temperature. Then, cyano sodium borohydride (16 mg, 0.2548 mmol) and acetone (0.045 ml, 0.6129mmol) were added, followed by further stirring for 4 hours at room temperature. After the addition of 2N hydrochloric acid (4 ml), the reaction mixture was made alkaline with dilute sodium hydroxide aqueous solution. Methanol was evaporated under reduced pressure. The water layer was extracted with ethyl acetate. The organic layer was washed with water, then with saturated brine, and dried with anhydrous sodium sulfate. The solvent was evaporated under reduced pressure. The resulting residue was submitted to basic silica gel column chromatography (hexane:ethyl acetate = 4:1). A powder of isopropyl[3-(3-pyridyl) benzo[b]thiophen-6-yl]amine (101mg, 73%) was obtained by crystallizing in petroleum ether.
$^1$H-NMR (DMSO-$d_6$) δ: 1.24 (d, J=6.1Hz, 6H), 3.67 (br s, 1H), 3.69 (sept, J=6.1Hz, 1H), 6.67 (dd, J=2.1, 8.9Hz, 1H), 7.02 (d, J=2.1Hz, 1H), 7.09(s, 1H), 7.36 (dd, J=4.9, 7.6Hz, 1H), 7.57 (d, J=8.9Hz, 1H), 7.85 (dt, J=1.8, 7.9Hz, 1H), 8.59 (dd, J=1.5, 4.9Hz, 1H), 8.81 (d, J=1.5Hz, 1H).
IR (KBr) : 3329, 2965, 1606, 1561, 1525, 1482, 1460, 1334, 1254, 817, 755, 739, 714 $cm^{-1}$.

Example 55

Preparation of 4-[3-(3-pyridyl)benzo[b]thiophen-6-yl] morpholine dihydrochloride

Under nitrogen atmosphere, a solution of 3-(3-pyridyl) benzo[b]thiophen-6-yl=trifluoromethane sulfonate (241 mg, 0.6707 mmol) obtained in Example 13 in toluene (2 ml) and morpholine (88 μl, 1.009 mmol) were added to suspension of tris(dibenzylideneacetone)dipalladium (0) (31 mg, 0.03385 mmol), 1,1'-bis(diphenylphosphino)ferrocene (37 mg, 0.0674 mmol), and sodium t-butoxide (97 mg, 1.009 mmol) in toluene (5 ml). The mixture was reacted for 5.5 hours at 100°C. The reaction mixture was cooled, diluted with ethyl acetate, washed with water, and extracted with 2N hydrochloric acid. The water layer was made alkaline with dilute sodium hydroxide aqueous solution, then extracted with ethyl acetate. The organic layer was washed with saturated brine, and dried with anhydrous sodium sulfate. The solvent was evaporated under reduced pressure. The resulting residue was submitted to basic silica gel column chromatography (hexane:diethyl ether = 1:1). A powder of 4-[3-(3-pyridyl)benzo[b]thiophen-6-yl]morpholine dihydrochloride (73 mg, 29%) was obtained from the eluate by crystallizing in 1N hydrogen chloride-diethyl ether.
$^1$H-NMR (DMSO-$d_6$) δ: 3.25 (t, J=4.9Hz, 4H), 3.81 (t, J=4.9Hz, 4H), 7.32(d, J=9.2Hz, 1H), 7.71 (br s, 1H), 7.82 (d, J=9.2Hz, 1H), 8.00 (s, 1H), 8.12 (dd, J=5.5, 7.3Hz, 1H), 8.75 (d, J=7.3Hz, 1H), 8.90 (d, J=5.5Hz, 1H), 9.14 (s, 1H).
IR (KBr): 3263, 3062, 2559, 1559, 1455, 1405, 1317, 1321, 1266, 1118, 1055, 910, 795, 679, 620 $cm^{-1}$.

Example 56

Preparation of Nl-[3-(3-pyridyl)benzo[b]thiophen-6-yl] acetamide

Acetic anhydride (0.5 ml) was added to a solution of 3-(3-pyridyl)benzo[b]thiophen-6-yl amine (100 mg, 0.4419 mmol) obtained in Example 54 in pyridine (1 ml), and the mixture was stirred for 45 minutes at room temperature. The reaction mixture was poured into ice water. The precipitate formed was filtered and washed with ethyl acetate, then with diethyl ether to obtain a pale red powder of N1-[3-(3-pyridyl)benzo[b]thiophen-6-yl] acetamide (60 mg, 51%).
$^1$H-NMR ($CDCl_3$) δ: 2.22 (s, 3H), 7.25 (m, 1H), 7.38 (s, 1H), 7.40 (dd, J=4.9, 7.9Hz, 1H), 7.73 (d, J=8.9Hz, 1H), 7.85 (m, 1H), 8.42(m, 1H), 8.62 (m, 1H), 8.81 (m, 1H).
IR (KBr): 3282, 3085, 1661, 1582, 1539, 1469, 1397, 1371, 1334, 1280, 800, 712 $cm^{-1}$.

Example 57

The measurement of steroid 17α-hydroxylase and/or steroid C17-20 lyase inhibitory activity

An experiment was carried out according to the method of T. Sergejew and R. W. Hartmann (J. Enzyme

Inhibition, 8, 113(1994)). That is, the testis of rats (SD, male) was homogenized and centrifuged to obtain microsome. Each compound of the present invention prepared in Examples 1-56 was put into a micro tube (1.5 ml, Eppendorf Co.). After the addition of 100 μl of microsome protein solution, the protein contentration was adjusted to 0.1 mg/ml by 50 mM phosphate buffer solution (pH 7.4), 140 μl of 125 nmol NADPH solution, and 10 μl of 6.25 nmol 17α-hydroxyprogesterone, the mixture was incubated for 20 minutes at 37°C. 50 μl of 1N hydrochloric acid and 1000 μl of ethyl acetate were sequentially added to the mixture. The mixture was shaken and centrifuged. The ethyl acetate layer was washed with 250 μl of 50 mM phosphate buffer solution (pH 7.4) and 50 μl of 1N hydrochloric acid, centrifuged, and concentrated. The concentrate was dissolved in 100 μl of acetonitrile. 10 μl of this solution was charged to high performance liquid chromatography. The amounts of the substrate and the product (androstenedione and testosterone) were measured to calculate the enzyme activity. In this experiment, a sample without the test compound was provided as a control group. Steroid 17α-hydroxylase and/or steroid $C_{17-20}$-lyase inhibitory activity (%) was calculated by the following equation (1) using the amounts of the substrate and the product obtained. Main results are shown in Table 1.

Inhibitory activity (%)

$$= 100 - \frac{\text{(Enzymatic activity with test compound)}}{\text{(Enzymatic activity without test compound)}} \quad (1)$$

TABLE 1

| Example | Inhibitory activity (%) |
|---|---|
| 2 | 89 |
| 3 | 9 |
| 6 | 3 |
| 7 | 60 |
| 8 | 59 |
| 9 | 63 |
| 10 | 71 |
| 11 | 71 |
| 12 | 100 |
| 13 | 93 |
| 14 | 91 |
| 15 | 93 |
| 16 | 73 |
| 17 | 100 |
| 18 | 37 |
| 19 | 68 |
| 20 | 100 |
| 21 | 30 |
| 22 | 73 |
| 23 | 100 |
| 24 | 83 |
| 25 | 76 |
| 26 | 1 |
| 27 | 56 |
| 28 | 42 |
| 29 | 25 |
| 30 | 100 |
| 31 | 100 |
| 32 | 100 |
| 33 | 100 |
| 34 | 100 |
| 35 | 61 |
| 36 | 60 |

TABLE 1 (continued)

| Example | Inhibitory activity (%) |
|---|---|
| 37 | 80 |
| 38 | 45 |
| 39 | 64 |
| 40 | 79 |
| 41 | 95 |
| 42 | 57 |
| 43 | 97 |
| 44 | 46 |
| 45 | 33 |
| 46 | 100 |
| 47 | 53 |
| 48 | 54 |
| 49 | 67 |
| 50 | 80 |
| 51 | 37 |
| 52 | 93 |
| 53 | 86 |
| 54 | 98 |
| 55 | 94 |
| 56 | 63 |

Enzyme source: Rat testis microsome
Test compound concentration: 300 nM
Substrate concentration: 25 μM (17α-hydroxyprogesterone)
NADPH concentration: 500 μM

INDUSTRIAL APPLICABILITY

Novel benzothiophene derivatives are provided by the present invention. The compounds of the present invention exhibit potent inhibitory activity of steroid 17α-hydroxylase and/or steroid C17-20 lyase. They also inhibit aromatase. Due to their activity, the compounds of the present invention are useful as preventive and/or therapeutic agents for various diseases depending upon androgenic hormones and estrogens, such as prostate cancer, prostatic hypertrophy (prostatism), androgenic syndrome (masculinization), andromorphous baldness, breast cancer, mastopathy, uterine cancer, endometriosis, and ovarian cancer.

## Claims

**1.** Benzothiophene derivatives represented by the following formula (I) or salts thereof:

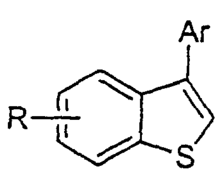

(I)

wherein Ar is a substituted or unsubstituted aromatic heterocyclic group; and R is a hydroxyl group, lower alkyl group, lower alkyloxy group, halogen atom, carboxyl group, lower alkyloxycarbonyl group, carbamoyl group, morpholino group, amino group, amino group which may be substituted or not substituted with one or more substituents selected from a lower alkyl group and lower acyl group, cyano group, substituted or unsubstituted phenyl group,

substituted or unsubstituted phenoxy group, substituted or unsubstituted phenyl lower alkyl group, substituted or unsubstituted phenyl lower alkyloxy group, or substituted or unsubstituted aromatic heterocyclic group; wherein the lower alkyl group is a hydrocarbon having 1-7 carbon atoms which may be linear, branched, or cyclic, and the hydrocarbon may be substituted with a halogen atom, hydroxyl group, alkyloxy group, amino group, amino group which may be substituted or not substituted with one or two substituents selected from a lower alkyl group and lower acyl group, nitro group, or cyano group; the halogen atom shows a fluorine atom, chlorine atom, bromine atom, or iodine atom; as the substituent in the substituted phenyl group, substituted phenoxy group, substituted phenyl lower alkyl group, or substituted phenyl lower alkoxy group of R, or in the substituted aromatic heterocyclic group of Ar and R, a hydroxyl group, lower alkyl group, lower alkylcarbonyl group, lower alkyloxy group, lower alkylthio group, halogen atom, carboxyl group, lower alkyloxycarbonyl group, carbamoyl group, amino group, amino group which may be substituted or not substituted with one or two substituents selected from a lower alkyl group and a lower acyl group, nitro group, and cyano group can be given, wherein the number of substituent may be 1-3 and the two substituents in combination may form a lower alkylenedioxy group.

**2.** Inhibitors of steroid 17α-hydroxylase and/or C17-20 lyase comprising a benzothiophene derivative or salt according to claim 1.

**3.** Pharmaceutical compositions comprising a benzothiophene derivative or salt according to claim 1.

## INTERNATIONAL SEARCH REPORT

International application No.
PCT/JP01/04189

A. CLASSIFICATION OF SUBJECT MATTER
Int.Cl[7] C07D409/04, 417/04, A61K31/4436, 31/427, A61P5/28, 5/32, 35/00

According to International Patent Classification (IPC) or to both national classification and IPC

B. FIELDS SEARCHED

Minimum documentation searched (classification system followed by classification symbols)
Int.Cl[7] C07D409/04, 417/04, A61K31/4436, 31/427, A61P5/28, 5/32, 35/00

Documentation searched other than minimum documentation to the extent that such documents are included in the fields searched

| | | | |
|---|---|---|---|
| Jitsuyo Shinan Koho | 1926-1996 | Toroku Jitsuyo Shinan Koho | 1994-2001 |
| Kokai Jitsuyo Shinan Koho | 1971-2001 | Jitsuyo Shinan Toroku Koho | 1996-2001 |

Electronic data base consulted during the international search (name of data base and, where practicable, search terms used)
CAPLUS (STN)
REGISTRY (STN)

C. DOCUMENTS CONSIDERED TO BE RELEVANT

| Category* | Citation of document, with indication, where appropriate, of the relevant passages | Relevant to claim No. |
|---|---|---|
| X | WO 99/07700 A1 (SMITHCLINE BEECHAM PLC), 18 February, 1999 (18.02.99), Full text; esp., Descriptions 14,15 & EP 1003738 A1 | 1 |
| X | US 5846982 A (ELI LILLY AND COMPANY), 08 December, 1998 (08.12.98), Full text; esp., Claims 1 & WO 97/47302 A1 | 1,3 |
| X | EP 217530 A1 (ELI LILLY AND COMPANY), 08 April, 1987 (08.04.87), Full text; esp., working examples 29,30 & JP 62-45586 A | 1,3 |
| A | WO 95/24406 A1 (YAMANOUCHI PHARMACEUTICAL CO., LTD.), 14 September, 1995 (14.09.95), Full text; esp., working example 12 & JP 7-523359 A | 1,3 |
| A | EP 445073 A1 (CIBA-GEIGY AG), 04 September, 1991 (04.09.91), | 1-3 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>09 July, 2001 (09.07.01) | Date of mailing of the international search report<br>24 July, 2001 (24.07.01) |
| Name and mailing address of the ISA/<br>Japanese Patent Office<br>Facsimile No. | Authorized officer<br>Telephone No. |

Form PCT/ISA/210 (second sheet) (July 1992)

INTERNATIONAL SEARCH REPORT

International application No.
PCT/JP01/04189

C (Continuation). DOCUMENTS CONSIDERED TO BE RELEVANT

| Category* | Citation of document, with indication, where appropriate, of the relevant passages | Relevant to claim No. |
|---|---|---|
| | Full text<br>& JP 4-211684 A | |

Form PCT/ISA/210 (continuation of second sheet) (July 1992)